# EUROPEAN PATENT SPECIFICATION

(11) **EP 3 424 853 B1**
(45) Date of publication and mention of the grant of the patent: **12.02.2020**
(21) Application number: 16892456.1
(22) Date of filing: 29.02.2016
(51) Int. Cl.: B65H 19/20, A61F 13/15, A61F 13/49

(54) **MATERIAL SPLICING METHOD, AND MATERIAL SUPPLY DEVICE**
MATERIALSPLEISSVERFAHREN UND MATERIALZUFÜHRVORRICHTUNG
PROCÉDÉ D'ÉPISSAGE DE MATÉRIAU, ET DISPOSITIF D'APPORT DE MATÉRIAU

(43) Date of publication of application: 09.01.2019
(73) Proprietor: Unicharm Corporation, Shikokuchuo-shi, Ehime 799-0111 (JP)
(72) Inventor: ISHIKAWA, Yoshihide, Kanonji-shi Kagawa 769-1602 (JP); TADA, Hiroaki, Kanonji-shi Kagawa 769-1602 (JP)
(74) Representative: Staeger & Sperling Partnerschaftsgesellschaft mbB
(86) International application number: PCT/JP2016/056099
(87) International publication number: WO 2017/149611

(56) References cited:
- JP-A- H05 270 706
- JP-A- H05 286 615
- JP-A- H10 203 698
- JP-A- 2003 128 309
- JP-A- 2014 012 599
- JP-A- 2015 157 450
- US-A- 5 643 395
- US-A- 5 643 395

## Description

### [Technical Field]

The invention relates to a method for splicing a material and a device for supplying a material.

### [Background Art]

Conventionally known is a method for splicing a material associated with an absorbent article including: transporting the material in the direction of transport; and when the material being transported is defined as a preceding material, splicing a subsequent material with the preceding material by bonding the leading end portion of the subsequent material with that preceding material.

The material is transported in the direction of transport while being wound around an entrance roll and a moving roll of a dancer unit. The transport of the material is controlled so that the moving roll is positioned at a reference position.

### [Citation List]

### [Patent Literature]

[PTL 1] European Patent Application Publication No. 2491 909. Further prior art in this technical field is disclosed in documents JP H10 203698 A and US 5,643,395 A.

### [Summary of Invention]

### [Technical Problem]

The leading end portion of the subsequent material is bonded with the preceding material, forming a bonded portion. Then, as the material is transported, the bonded portion then reaches the moving roll. There is a problem that, when the bonded portion reaches the moving roll, the material is impacted on, causing fluttering (rampage) of the material and variation in tension in the material.

The invention has been made in view of the above problems, and an advantage thereof is to suppress variation in tension in a material.

### [Solution to Problem]

An aspect of the invention to achieve the above advantage is a method for splicing a material, including:
transporting the material in a direction of transport while the material being wound around an entrance roll and a moving roll,
   the entrance roll and the moving roll included in a dancer unit;
splicing a subsequent material with a preceding material by bonding a leading end portion of the subsequent material with the preceding material,
   the preceding material being the material that is transported;
forming a fin-like portion of the preceding material upstream in the direction of transport from a bonded portion where the preceding material and the subsequent material are bonded,
   the forming being performed by cutting the preceding material at a position upstream from the bonded portion;
continuing transport of the material in which the bonded portion and a stacking portion are provided side-by-side along the direction of transport, while the material being wound around the entrance roll and the moving roll,
   the stacking portion being a portion where the fin-like portion and the subsequent material are stacked; and
controlling transport of the material so that the moving roll is located at a reference position,
in the forming the fin-like portion,
   the preceding material is cut so that a total length of the bonded portion and the stacking portion is larger than a path length of the material from a downstream end of a winding portion of the material to an upstream end of another winding portion of the material,
   the winding portion being a portion wound around the entrance roll,
   the other winding portion being a portion wound around the moving roll located at the reference position.

Other features of this invention will become apparent from the description in this specification and the attached drawings.

### [Advantageous Effects of Invention]

According to the invention, it is possible to suppress variation in tension in a material.

### [Brief Description of Drawings]

[FIG. 1] FIG. 1A is a schematic side view of a manufacturing line LM of a disposable diaper 1 exemplifying an absorbent article, and FIG. 1B is a schematic plan view of the manufacturing line LM along arrows B-B in FIG. 1A.
[FIG. 2A] FIG. 2A is a view along arrows IV-IV in FIG. 1B.
[FIG. 2B] FIG. 2B is a schematic plan view along arrows B-B in FIG. 2A.
[FIG. 3] FIG. 3 is a diagram schematically showing the state of a material 3 when cutting a preceding material 3a.
[FIG. 4] FIG. 4 is a diagram schematically showing the state of a material 3 when a belt member 26F moves back to a stand-by position.
[FIG. 5] FIG. 5 is a diagram showing Positions P1 to P7 of a material 3 in its transport path and path lengths of L12 to L67 of the material 3 between these Positions.
[FIG. 6] FIG. 6 is a first diagram illustrating advantages (effects) of the present embodiment.
[FIG. 7] FIG. 7 is a second diagram illustrating advantages (effects) of the present embodiment.
[FIG. 8] FIG. 8 is a third diagram illustrating advantages (effects) of the present embodiment.
[FIG. 9] FIG. 9 is a fourth diagram illustrating advantages (effects) of the present embodiment.
[FIG. 10] FIG. 10 is a fifth diagram illustrating advantages (effects) of the present embodiment.
[FIG. 11] FIG. 11 is a sixth diagram illustrating advantages (effects) of the present embodiment.
[FIG. 12] FIG. 12 is a diagram showing the first modified example associated with a pressing mechanism.
[FIG. 13] FIG. 13 is a diagram showing the second modified example associated with a pressing mechanism.

### [Description of Embodiments]

At least the following matters will be made clear by the description in the present specification and the accompanying drawings.

A method for splicing a material associated with an absorbent article, including:
transporting the material in a direction of transport while the material being wound around an entrance roll and a moving roll,
   the entrance roll and the moving roll included in a dancer unit;
splicing a subsequent material with a preceding material by bonding a leading end portion of the subsequent material with the preceding material,
   the preceding material being the material that is transported;
forming a fin-like portion of the preceding material upstream in the direction of transport from a bonded portion where the preceding material and the subsequent material are bonded,
   the forming being performed by cutting the preceding material at a position upstream from the bonded portion;
continuing transport of the material in which the bonded portion and a stacking portion are provided side-by-side along the direction of transport, while the material being wound around the entrance roll and the moving roll,
   the stacking portion being a portion where the fin-like portion and the subsequent material are stacked; and
controlling transport of the material so that the moving roll is located at a reference position,
in the forming the fin-like portion,
   the preceding material is cut so that a total length of the bonded portion and the stacking portion is larger than a path length of the material from a downstream end of a winding portion of the material to an upstream end of another winding portion of the material,
   the winding portion being a portion wound around the entrance roll,
   the other winding portion being a portion wound around the moving roll located at the reference position.

With such a method for splicing a material, it is possible to suppress variation in tension in the material.

In such a method for splicing a material, it is desirable that
in the forming the fin-like portion,
the preceding material is cut so that a length of the stacking portion is larger than a path length of the material from the downstream end of the winding portion of the material to a downstream end of the other winding portion of the material,
the winding portion being a portion wound around the entrance roll,
the other winding portion being a portion wound around the moving roll located at the reference position.

With such a method for splicing a material, it is possible to suppress variation in tension in the material more appropriately.

In such a method for splicing a material, it is desirable
that the material is transported in the direction of transport while the material being wound around the entrance roll, the moving roll, and an exit roll of the dancer unit,
that after splicing the subsequent material and the preceding material,
   transport of the material in which the bonded portion and the stacking portion are provided side-by-side along the direction of transport continues while the material being wound around the entrance roll, the moving roll, and the exit roll, and
that, in the forming the fin-like portion,
   the preceding material is cut so that the total length of the bonded portion and the stacking portion is larger than a path length of the material from the downstream end of the winding portion of the material, through the moving roll located at the reference position, to an upstream end of another winding portion of the material,
   the winding portion being a portion wound around the entrance roll,
   the other winding portion being a portion wound around the exit roll.

With such a method for splicing a material, it is possible to suppress variation in tension in the material more appropriately.

In such a method for splicing a material, it is desirable that
in the forming the fin-like portion,
the preceding material is cut so that the length of the stacking portion is larger than a path length of the material from the downstream end of the winding portion of the material, through the moving roll located at the reference position, to a downstream end of the other winding portion of the material,
the winding portion being a portion wound around the entrance roll,
the other winding portion being a portion wound around the exit roll.

With such a method for splicing a material, it is possible to suppress variation in tension in the material more appropriately.

In such a method for splicing a material, it is desirable
that the material is transported in the direction of transport while the material being wound around the entrance roll, the moving roll, and an exit roll of the dancer unit,
that, after splicing the subsequent material and the preceding material,
   transport of the material in which the bonded portion and the stacking portion are provided side-by-side along the direction of transport continues while the material being wound around the entrance roll, the moving roll, and the exit roll, and
that, in the forming the fin-like portion,
   the preceding material is cut so that the total length of the bonded portion and the stacking portion is smaller than a path length of the material from the downstream end of the winding portion of the material, through the moving roll located at the reference position, to an upstream end of another winding portion of the material,
   the winding portion being a portion wound around the entrance roll,
   the other winding portion being a portion wound around the exit roll.

With such a method for splicing a material, it is possible to suppress variation in tension in the material and also suppress adverse effect by elongation of the fin-like portion.

In such a method for splicing a material, it is desirable that
in the forming the fin-like portion,
the preceding material is cut so that the total length of the bonded portion and the stacking portion is larger than a path length of the material from an upstream end of the winding portion of the material to the upstream end of the other winding portion of the material,
the winding portion being a portion wound around the entrance roll,
the other winding portion being a portion wound around the moving roll located at the reference position.

With such a method for splicing a material, it is possible to suppress variation in tension in the material more appropriately.

In such a method for splicing a material, it is desirable
that, when bonding the subsequent material with the preceding material being transported,
   the preceding material is pressed against the subsequent material by moving an abutting member from a stand-by position to an abutting position where the abutting member abuts on the preceding material,
that, after the bonded portion of the material being transported has reached the moving roll,
   the abutting member is moved back from the abutting position to the stand-by position, and
that, in the forming the fin-like portion,
   the preceding material is cut so that the total length of the bonded portion and the stacking portion is larger than a path length of the material from a downstream end of an abutting portion of the material to the upstream end of the other winding portion of the material,
   the abutting portion being a portion that abuts on the abutting member,
   the other winding portion being a portion wound around the moving roll located at the reference position.

With such a method for splicing a material, it is possible to suppress variation in tension in the material more appropriately.

In such a method for splicing a material, it is desirable
that, after the bonded portion of the material being transported has passed the moving roll,
   the abutting member is moved back from the abutting position to the stand-by position, and
that, in the forming the fin-like portion,
   the preceding material is cut so that a length of the stacking portion is larger than a path length of the material from the downstream end of the abutting portion of the material to the upstream end of the other winding portion of the material to a downstream end of the other winding portion of the material,
   the abutting portion being a portion that abuts on the abutting member,
   the other winding portion being a portion wound around the moving roll located at the reference position.

With such a method for splicing a material, it is possible to suppress variation in tension in the material more appropriately.

In such a method for splicing a material, it is desirable
that, when bonding the subsequent material with the preceding material being transported,
   the preceding material is pressed against the subsequent material by moving an abutting member from a stand-by position to an abutting position where the abutting member abuts on the preceding material, and
that, in the forming the fin-like portion,
   the preceding material is cut so that the total length of the bonded portion and the stacking portion is smaller than a path length of the material from a downstream end of an abutting portion of the material to the upstream end of the other winding portion of the material,
   the abutting portion being a portion that abuts on the abutting member,
   the other winding portion being a portion wound around the moving roll located at the reference position.

With such a method for splicing a material, it is possible to suppress variation in tension in the material and also suppress adverse effect by elongation of the fin-like portion.

In such a method for splicing a material, it is desirable that
that, when bonding the subsequent material with the preceding material being transported,
   the preceding material is pressed against the subsequent material by moving an abutting member from a stand-by position to an abutting position where the abutting member abuts on the preceding material, and
that the preceding material is cut with the abutting member being located at the abutting position.

With such a method for splicing a material, it is possible to suppress variation in tension in the material more appropriately.

In such a method for splicing a material, it is desirable that
after releasing the abutting of the fin-like portion of the material being transported on the abutting member,
the abutting member is moved back from the abutting position to the stand-by position.

With such a method for splicing a material, it is possible to suppress variation in tension in the material more appropriately.

In such a method for splicing a material, it is desirable
that, when bonding the subsequent material with the preceding material being transported,
   the preceding material is pressed against the subsequent material by moving an abutting member from a stand-by position to an abutting position where the abutting member abuts on the preceding material, and
that the abutting member is a belt member being capable of rotating.

With such a method for splicing a material, it is possible to suppress variation in tension in the material more appropriately.

In such a method for splicing a material, it is desirable that
the belt member is a member
that rotates due to rotatings of a first rotation roller and a second rotation roller while the belt member being wound around the first rotation roller and the second rotation roller, and
that moves from the stand-by position to the abutting position by swinging about a swing axis,
   the swing axis being a central axis of the first rotation roller.

With such a method for splicing a material, it is possible to suppress variation in tension in the material more appropriately.

In such a method for splicing a material, it is desirable
that the material is a continuous sheet being a fiber assembly,
that one surface of the continuous sheet has a density of fiber higher than another surface of the continuous sheet, and
that, when bonding the subsequent material with the preceding material being transported,
   the preceding material is pressed against the subsequent material while an adhesive member being sandwiched between the preceding material and the subsequent material by moving an abutting member from a stand-by position to an abutting position where the abutting member abuts on the one surface of the preceding material.

With such a method for splicing a material, it is possible to suppress penetration of an adhesive member (the adhesive member itself or adhesive provided by the adhesive member).

In such a method for splicing a material, it is desirable
that the material is a continuous sheet being a fiber assembly,
that one surface of the continuous sheet has a density of fiber higher than another surface of the continuous sheet, and
that, in the forming the fin-like portion by cutting the preceding material,
   a cutter blade enters from the one surface.

With such a method for splicing a material, it is possible to easily cut the preceding material.

Next, a device for supplying a material associated with an absorbent article including:
a dancer unit including an entrance roll and a moving roll;
a transport section that transports the material in a direction of transport while the material wound around the entrance roll and the moving roll;
a material splicing section that splices a subsequent material with a preceding material by bonding a leading end portion of the subsequent material with the preceding material,
   the preceding material being the material that is transported;
a cutting section that forms a fin-like portion of the preceding material upstream in the direction of transport from a bonded portion where the preceding material and the subsequent material are bonded,
   the forming being performed by cutting the preceding material at a position upstream from the bonded portion,
   transport of the material in which the bonded portion and a stacking portion are provided side-by-side along the direction of transport being continued by the transport section while the material being wound around the entrance roll and the moving roll,
   the stacking portion being a portion where the fin-like portion and the subsequent material are stacked; and
a control section that controls transport of the material so that the moving roll is located at a reference position,
the cutting section cutting the preceding material so that a total length of the bonded portion and the stacking portion is larger than a path length of the material from a downstream end of a winding portion of the material to an upstream end of another winding portion of the material,
   the winding portion being a portion wound around the entrance roll,
   the other winding portion being a portion wound around the moving roll located at the reference position.

With such a device for supplying a material, it is possible to suppress variation in tension in the material.

### = The Present Embodiment =

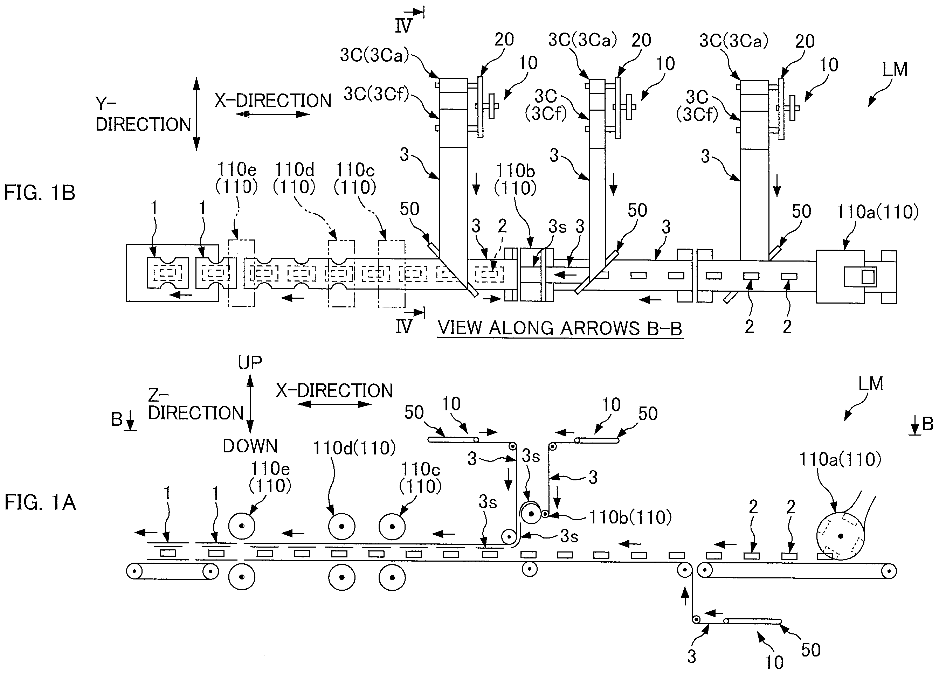

FIG. 1A is a schematic side view of the manufacturing line LM of an absorbent article, and FIG. 1B is a schematic plan view of the manufacturing line LM along arrows B- B in FIG. 1A. Note that there are cases where normally visible members have been omitted from FIGS. 1A and 1B in order to prevent complication in the drawings.

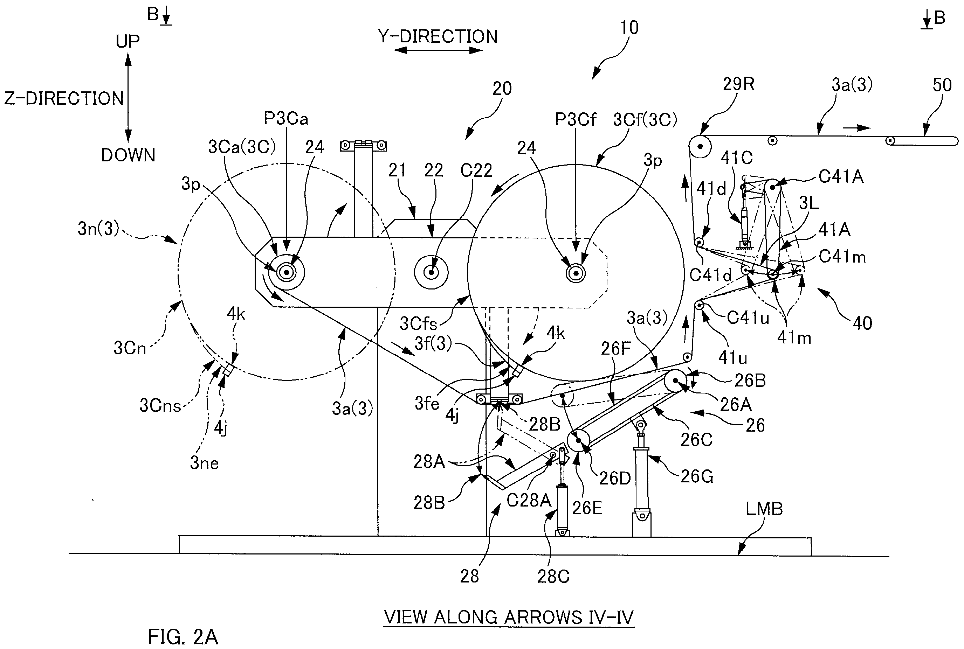

In the manufacturing line LM, a disposable diaper 1 is manufactured as an example of an absorbent article. In the manufacturing line LM, a plurality of continuous sheets 3, 3,... (which are fiber assemblies) are used as a material 3. For example, used are a plurality of soft and flexible continuous sheets 3 such as nonwoven fabric and tissue paper. The materials 3, 3,... are brought into the manufacturing line LM in the form of respective material coils 3C in each of which the continuous sheet 3 (the material) is wound around a paper tube 3p (FIG. 2A) in a coil-like manner. As for the continuous sheet 3, the density of fiber of its top surface (corresponding to the one surface) is higher than the density of fiber of its back surface (corresponding to the other surface). The continuous sheet 3 is wound in the material coil 3C so that the top surface becomes the outer circumferential surface of the material coil 3C and the back surface becomes the inner circumferential surface of the material coil 3C.

Various types of the material coils 3C, 3C,... are mounted to material supplying devices 10 provided in the manufacturing line LM for the respective types of materials 3, and thus the materials 3 are fed. While being transported along predetermined transport paths in the manufacturing line LM, the materials 3 are subjected to processing such as pressing and cutting by various types of processing units 110, 110,... (processing devices), and further combined, for example, other materials 3 and/or appropriate member 2, ultimately manufacturing the disposable diaper 1.

As examples of the processing units 110, there are provided a fiber-depositing device 110a, a cutting device 110b, a pressing device 110c, a leg-opening cutting device 110d, an end-cutting device 110e and the like. However, the invention is not limited thereto. The devices 110a, 110b, 110c, 110d and 110e have respective functions as follow, for example.

The fiber-depositing device 110a produces the absorbent body 2 that serves as the abovementioned member, the absorbent body 2 mainly made of liquid absorbent fiber such as pulp fiber.

The cutting device 110b cuts the material 3 into single-cut leak-proof sheets 3s, and transports the leak-proof sheets 3s while spacing between each pair of the leak-proof sheets 3s that are adjacent in the direction of transport. With keeping the spacing, each leak-proof sheet 3s is bonded to another material 3. Note that the cutting device 110b is exemplified by a commonly-known slip cutting device (e.g., Japanese Patent Application Publication No. 2011-083547).

The pressing device 110c presses various types of materials 3, 3,... by a pair of upper and lower rolls.

The leg-opening cutting device 110d forms leg openings in the materials 3, 3,... by a pair of upper and lower rolls.

The end-cutting device 110e cut out a single-piece disposable diaper 1 from the materials 3, 3,... by a pair of upper and lower rolls, producing the disposable diaper 1.

In the following description, three directions perpendicular to one another in the manufacturing line LM are respectively referred to as X-direction, Y-direction and Z-direction. As shown in FIG. 1B, X-direction and Y-direction are in the horizontal direction, and as shown in FIG. 1A, Z-direction is in the vertical direction. As shown in FIG. 1B, X-direction and Y-direction are perpendicular to each other.

In the manufacturing line LM, various types of the processing units 110, 110,... are arranged side-by-side along X-direction. Accordingly, between the processing units 110, 110,..., the material 3 is basically transported along X-direction as viewed from above.

As shown in FIG. 1B, in order to reduce the total length of the manufacturing line LM in X-direction, the material supplying devices 10 are each arranged at a position in Y-direction located away from the processing units 110, 110,... in the manufacturing line LM. Accordingly, the materials 3 are supplied mainly along Y-direction from the material supplying devices 10 to the processing units 110, 110,.... That is, after the material 3 which each material supplying device 10 has fed along Y-direction is transported along Y-direction, a turn bar 50 (to be described later) changes the direction of transport of the material 3 to X-direction, supplying the material 3 to the processing unit 110 corresponding thereto.

The foregoing material supplying devices 10 are provided corresponding to the types of the materials 3 as mentioned above, and the basic configurations of the material supplying devices 10, 10,... are identical. Accordingly, one of the material supplying devices 10 will be described below.

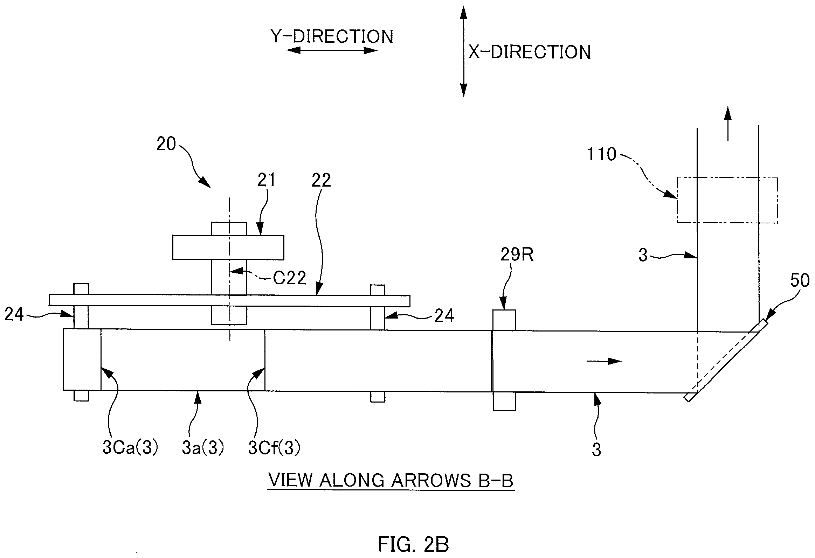

FIG. 2A is a view along arrows IV-IV in FIG. 1B. FIG. 2B is a schematic plan view along arrows B-B in FIG. 2A. In both of FIGS. 2A and 2B, some members are omitted for the purpose of preventing complication of the drawings.

The material supplying device 10 includes a material-splicing device 20. Before the preceding material 3a which is being fed from the material coil 3Ca runs out, the material-splicing device 20 bonds a subsequent material 3f with the material 3a; the subsequent material 3f is the material 3f of another material coil 3Cf which has not been fed yet. Thereby, the material 3 (3a, 3f) is supplied continuously to the processing unit 110 of the manufacturing line LM without interruption. Also, an accumulating device 40 is provided at a position downstream from the material-splicing device 20 in the direction of transport, and the accumulating device 40 accumulates the material 3 (3a, 3f) to be sent from the material-splicing device 20 in the form of a loop 3L. This suppresses variation in tension in the material 3 (3a, 3f). Further, the turn bar 50 is provided as a direction-of-transport changing member and is located downstream from the accumulating device 40 in the direction of transport. The turn bar 50 changes the direction of transport of the material 3 from Y-direction to X-direction, and consequently the material 3 whose direction of transport has been changed to X-direction is sent to the processing unit 110.

The devices and components associated with the material supplying device 10 will be described below.

The material-splicing device 20 includes: a support plate 21; a turret 22; two feeding rotation shafts 24 and 24 (corresponding to the transport section); servomotors (not shown); a pressing mechanism.26 (corresponding to the material splicing section); a cutter mechanism 28 (corresponding to the cutting section); and a controller (not shown). The support plate 21 is a plate such as a so-called panel board provided upright on the floor portion LMB of the manufacturing line LM. The turret 22 has an elongated plate shape and is supported by the support plate 21 in a manner of being capable of pivoting about the pivot axis C22 extending along X-direction. The two feeding rotation shafts 24 and 24 are provided on the longitudinal ends of the turret 22 and extend along X-direction. One of the servomotors is for driving and rotating the turret 22, and the others are each for driving and rotating respective one of the two feeding rotation shafts 24 and 24. The pressing mechanism 26 bonds the preceding material 3a with the subsequent material 3f by pressing the preceding material 3a against the outer circumferential surface 3Cfs of the subsequent-material coil 3Cf (that is, the top surface) when the preceding material 3a is being fed with one feeding rotation shaft 24; the subsequent-material coil 3Cf is supported by the other feeding rotation shaft 24. The cutter mechanism 28 cuts out the preceding material 3a from the paper tube 3p of the preceding-material coil 3Ca after the bonding. The controller is a computer or a sequencer that controls them.

The two feeding rotation shafts 24 and 24 are provided in a point symmetric relationship with respect to the pivot axis C22 of the turret 22. Accordingly, pivoting the turret 22 about the pivot axis C22 makes it possible to switch the positions of the rotation shafts 24 and 24. Each of the feeding rotation shafts 24 and 24 can support the material coil 3C by being inserted into the paper tube 3p at the center of the material coil 3C. The feeding rotation shaft 24 which is inserted into and supports the material coil 3C is driven and rotates, feeding the material 3 from the material coil 3C.

The two feeding rotation shafts 24 and 24 perform the feeding operation basically alternatingly. Specifically, while the one feeding rotation shaft 24 is feeding the material 3a from the material coil 3Ca, the other feeding rotation shaft 24 is in standby state of not performing feeding. When the material 3a of the one feeding rotation shaft 24 is about to run out, a preceding material 3a (the material 3a) is bonded with a subsequent material 3f; The subsequent material 3f is a material 3f of the material coil 3Cf which is attached to the other feeding rotation shaft 24 and which has not been fed yet. Accordingly, the other feeding rotation shaft 24 subsequently feeds and supplies the material 3f from the subsequent-material coil 3Cf. Furthermore, when the material 3f of the other feeding rotation shaft 24 is about to run out, the same operation as described above is performed again; at this stage, the material 3f becomes the preceding material, and an unfed material coil 3Cn newly attached to the one feeding rotation shaft 24 becomes the subsequent-material coil.

Also, in order to perform this bonding operation smoothly, a subsequent-material coil position P3Cf and a preceding-material coil position P3Ca are set in the direction of pivoting of the turret 22; the subsequent-material coil position P3Cf is a position at which the subsequent-material coil 3Cf which has not been fed yet is to be located during the bonding operation, and the preceding-material coil position P3Ca is a position at which the preceding-material coil 3Ca which is being fed is to be located during the bonding operation. In this example, the subsequent-material coil position P3Cf and the preceding-material coil position P3Ca are set on respective sides in Y-direction, with the same height in the up-down direction (Z-direction). However, the invention is not limited thereto. In this example, the feeding rotation shafts 24 rotate in the counter-clockwise direction, and the material coils 3Ca and 3Cf thus feed the materials 3a and 3f from below. For this reason, the transport path of the material 3a which is fed by the preceding-material coil 3Ca is defined below the subsequent-material coil 3Cf that is located at the subsequent-material coil position P3Cf. And, the pressing mechanism 26 and the cutter mechanism 28 are arranged further below the transport path.

The pressing mechanism 26 includes: a first fixed shaft 26A along X-direction; a first rotation roller 26B that rotates about the first fixed shaft 26A; a swinging arm 26C that swings about the first fixed shaft 26A; a second fixed shaft 26D that is provided on the end opposite to the first fixed shaft 26A of the swinging arm 26C; a second rotation roller 26E that rotates about the second fixed shaft 26D; an endless, belt member 26F (corresponding to the abutting member) that is provided in a manner of being capable of rotating and that is wound around the first rotation roller 26B and the second rotation roller 26E; an actuator 26G such as an air cylinder that swings the swinging arm 26C (a belt member 26F); and drive sources (not shown; e.g., servomotors) that drives the first rotation roller 26B or the second rotation roller 26E (the first rotation roller 26B in the present embodiment).

When bonding the subsequent material 3f with the preceding material 3a which is being transported, the belt member 26F that is located at a stand-by position is moved to an abutting position where the belt member 26F abuts on the preceding material 3a, pressing the preceding material 3a against the subsequent material 3f.

Specifically, the actuator 26G drives the swinging arm 26C, and thereby the swinging arm 26C swings. Accompanying with swinging of the swinging arm 26C, the second fixed shaft 26D and the second rotation roller 26E which is supported by the second fixed shaft 26D move toward the preceding material 3a. Then, the movement of the second rotation roller 26E moves the belt member 26F toward the preceding material 3a, and then the belt member 26F abuts on the preceding material 3a. Thus, the belt member 26F moves from the stand-by position to the abutting position by swinging about a swing axis, which is the central axis of the first rotation roller 26B (that is, the first fixed shaft 26A).

When moving the belt member 26F from the stand-by position to the abutting position, the preceding material 3a is being transported (to be described later in detail). Accordingly, when the belt member 26F abuts on the preceding material 3a, controlling is performed so that the belt member 26F is rotating at the same speed as the preceding material 3a in order for the preceding material 3a and the belt member 26F to move together. Specifically, when the belt member 26F moves from the stand-by position to the abutting position, the drive source drives the first rotation roller 26B and thereby the first rotation roller 26B rotates the belt member 26F by cooperating the second rotation roller 26E that follows the first rotation roller 26B. That is, the belt member 26F rotates by rotating the first rotation roller 26B and the second rotation roller 26E with the belt member 26F being wound around the first rotation roller 26B and the second rotation roller 26E.

After bonding is completed, the actuator 26G causes the swinging arm 26C (the belt member 26F) to swing in an inverse direction, moving the belt member 26F back from the abutting position to the stand-by position, and the drive source stops the rotating of the belt member 26F.

In the present embodiment, either one of the first rotation roller 26B or the second rotation roller 26E is a driving roller, causing the belt member 26F to be driven and to rotate. However, the invention is not limited thereto. The first rotation roller 26B and the second rotation roller 26E may be a follower roller, causing the belt member 26F to receive rotation force by coming into contact with the material 3.

The cutter mechanism 28 includes an arm member 28A, a cutter blade 28B and an actuator 28C (e.g., air cylinder). The arm member 28A is supported in a manner of being capable of swinging about the rotation axis C28A extending along X-direction. The cutter blade 28B is fixed to a swinging end of the arm member 28A. The actuator 28C drives the arm member 28A.

According to the swinging operation of the arm member 28A, the cutter blade 28B positioned at a stand-by position Pw28B moves toward and is brought into contact with the preceding material 3a from below, the preceding material 3a is cut. Thus, the preceding material 3a which has been bonded to the subsequent material 3f is cut and separated from the paper tube 3p of the feeding rotation shaft 24.

Note that a cutting position where the preceding material 3a is cut is located upstream in the direction of transport from an abutting portion on which the belt member 26F abuts. At the time of cutting, the cutter blade 28B enters from the foregoing top surface of the preceding material 3a (that is, a surface having a higher density of fiber). Accordingly, compared to the case where the cutter blade 28B enters from the back surface (a surface having a lower density of fiber), it is possible to easily cut the preceding material 3a (the preceding material 3a becomes easy to be cut).

The accumulating device 40 is a so-called dancer unit that accumulates, in the form of a loop 3L, the material 3 fed from the material-splicing device 20, so as to allow the material to be sent to the turn bar 50. By adjusting the size of the loop 3L, variation in tension in the material 3 is suppressed/absorbed (the tension in the material 3 is controlled), and the material 3 with suppressed variation in tension is sent to the turn bar 50.

The accumulating device 40 having this functionality includes: an entrance roll 41u and an exit roll 41d which are supported in a manner of being capable of rotating at respective fixed positions and which are located respectively on the entrance and the exit of the accumulating device 40; a moving roll 41m guided in a manner of being capable of moving back and forth in a predetermined direction (substantially Y-direction) in which the size of the loop 3L can be changed; an arm member 41A supported in a manner of being capable of swinging about the rotation axis C41A extending along X-direction so as to guide the moving roll 41m in a manner of being capable of moving back and forth in the predetermined direction. The entrance roll 41u, the moving roll 41m and the exit roll 41d are supported respectively in a manner of being capable of rotating about rotation axes C41u, C41m and C41d extending along X-direction. The material 3 is wound around (wound) around the entrance roll 41u, the moving roll 41m and the exit roll 41d in the following order in the direction of transport: the entrance roll 41u, the moving roll 41m and the exit roll 41d. Thus, the loop 3L is formed in the material 3. Further, the actuator 41C (e.g., air cylinder) applies a predetermined load (N) to the moving roll 41m via the arm member 41A in a direction for increasing the size of the loop 3L. Accordingly, if the tension (N) of the material 3 is smaller than a predetermined value based on a predetermined load, the moving roll 41m moves so as to increase the size of the loop 3L. On the other hand, if the tension (N) of the material 3 is larger than the predetermined value, the moving roll 41m moves so as to decrease the size of the loop 3L. The size of the loop 3L is measured by an appropriate sensor (not shown) such as a linear encoder or a rotary encoder, and a resulting measurement signal is transmitted to the controller (corresponding to the control section). Accordingly, based on this measurement signal, the controller corrects the instruction rotation speeds (rpm) of the feeding rotation shafts 24 and 24 of the material-splicing device 20 such that the size of the loop 3L is constant (in other words, such that the position of the moving roll 41m is stable), consequently controlling the tension in the material 3 appropriately (suppressing variation in tension). That is, the controller controls the transport of the material 3 so that the moving roll 41m is positioned at a predetermined position (hereinafter referred to as the reference position), suppressing variation in the size of the loop 3L. In the present embodiment, the center position of the positions of the three moving rolls 41m shown in FIG. 2A is defined as the reference position, and the other two positions are positions at which the size of the loop 3L is maximum (or minimum) .

In the process for correcting the instruction rotation speed, various correction methods can be used. As one example of correction process, the following process may be repeated at a predetermined control cycle. First, the actual value of the size of the loop 3L at the current time is obtained based on the measurement signal from the abovementioned sensor, and then a deviation amount is obtained by subtracting a target value for the size of the loop 3L from the actual value. Next, a control amount is calculated by multiplying the deviation amount by a predetermined control gain, and the control amount is subtracted from the abovementioned instruction rotation speed (rpm). Using the subtraction result as a corrected instruction rotation speed, the servomotor of the feeding rotation shaft 24 is controlled.

The correction process is not merely performed on the feeding rotation shaft 24 that feeds the preceding material 3a, but also performed on the feeding rotation shaft 24 that feeds the subsequent material 3f at least after bonding the subsequent material 3f with the preceding material 3a (at or after bonding). It is preferable that the correction process is performed immediately before bonding or when the rotation operation of the rotation shaft 24 starts. This makes it possible to reliably suppress variation in tension at the time of feeding of the first winding (first round) at the outer surface of the subsequent-material coil 3Cf.

The entrance roll 41u is a follower roll that is rotated due to rotation force received by coming into contact with the material 3. On the other hand, the exit roll 41d may likewise be a follower roll, or may be a driving roll that is driven and rotate due to driving rotation force received from a drive source such as a servomotor. In the present embodiment, the exit roll 41d is a follower roll, and a transport roll 29R (corresponding to the transport section) that is adjacent to the exit roll 41d on the downstream side in the direction of transport is a driving roll. Accordingly, in the present embodiment, the transport roll 29R and the feeding rotation shaft 24 cooperate and driving force from a servomotor is applied to each of them, transporting the material 3 in the direction of transport.

As shown in FIGS. 2A and 2B, the turn bar 50 changes the direction of transport of the material 3 sent by the accumulating device 40 from Y-direction to X-direction, sending the material 3 to the processing unit 110. As this turn bar 50, a round bar having a predetermined diameter, such as a stainless steel polished rod, is used, for example. That is, as shown in FIG. 2B, this round bar 50 is arranged in a immovable and unrotatable manner with its longitudinal direction being in a direction that is tilted by an angle of 45 degrees relative to both X-direction and Y-direction. Accordingly, when the material 3 is wrapped around the turn bar 50, the direction of transport of the material 3 is changed 90 degrees from Y-direction to X-direction.

### <<< Operations associated with Splicing of Material >>>

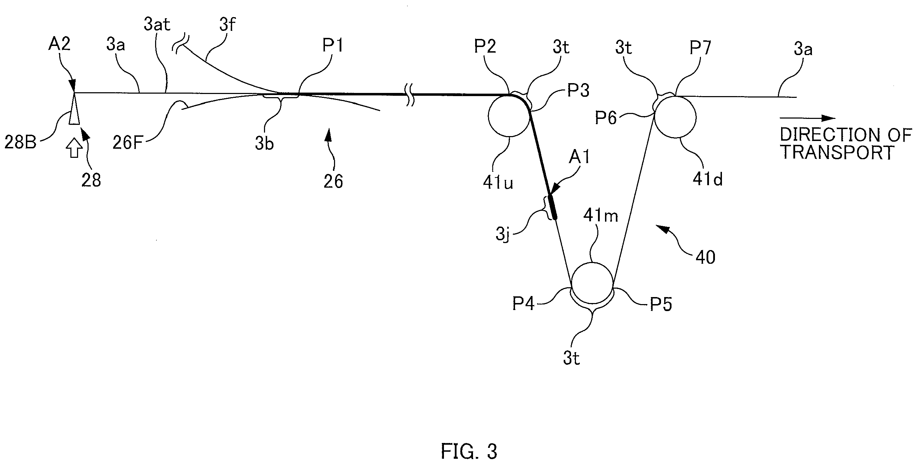

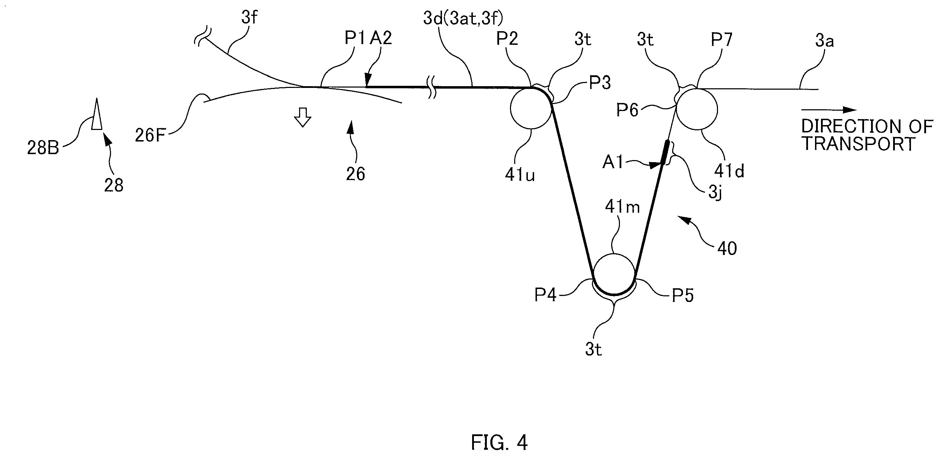

Next, operations of the manufacturing line LM (the material supplying device 10) when splicing materials will be described with reference to FIGS. 2A, 3, and 4. FIG. 3 is a diagram schematically showing the state of the material 3 when cutting the preceding material 3a. FIG. 4 is a diagram schematically showing the state of the material 3 when the belt member 26F moves back to the stand-by position.

At a time before the material splicing process starts, the material 3 is wound around rolls (the entrance roll 41u, the moving roll 41m, the exit roll 41d, the transport roll 29R, etc.) and is being transported in the direction of transport by the transport section (the transport roll 29R and the feeding rotation shaft 24).

At this stage, double-sided tape 4j for bonding (corresponding to the adhesive member) has already been provided on the leading end portion 3fe of the material 3f that is located on the outer circumferential surface 3Cfs of the subsequent-material coil 3Cf, and double-sided tape 4k for provisional retaining is provided on the back surface of the leading end portion 3fe such that the leading end portion 3fe does not become separated from the material coil 3Cf.

If the controller determines that the remaining amount of the material 3a of preceding-material coil 3Ca is equal to or less than a prescribed value, the controller starts the material splicing process. Specifically, the controller starts a process of bonding the leading end portion 3fe of the subsequent material 3f with the preceding material 3a (which is the material 3 being currently transported), splicing the subsequent material 3f with the preceding material 3a.

The controller controls the feeding rotation shaft 24, starting rotation of the subsequent-material coil 3Cf. And, the controller accelerates the subsequent-material coil 3Cf until the feeding speed of the subsequent material 3f becomes equal to the feeding speed of the preceding material 3a. Then, when the leading end portion 3fe has reached a position immediately before the belt member 26F, the controller controls the actuator 26G of the pressing mechanism 26, moving the belt member 26F from the stand-by position to the abutting position. That is, the pressing mechanism 26 moves the belt member 26F from the stand-by position to the abutting position, pressing the preceding material 3a against the subsequent material 3f. During this pressing, the leading end portion 3fe of the subsequent-material coil 3Cf passes the belt member 26F, and therefore the leading end portion 3fe and the preceding material 3a are bonded with the double-sided tape 4j. That is, the preceding material 3a is pressed against the subsequent material 3f while the double-sided tape 4j being sandwiched between the preceding material 3a and the subsequent material 3f, bonding the preceding material 3a and the subsequent material 3f without stopping the feeding operation.

In the present embodiment, when bonding the subsequent material 3f with the preceding material 3a which is being transported, the preceding material 3a is pressed against the subsequent material 3f while the double-sided tape 4j being sandwiched between the preceding material 3a and the subsequent material 3f by moving the belt member 26F from the stand-by position to the abutting position where the belt member 26F abuts on the top surface of the preceding material 3a (that is, a surface having a higher density of fiber). Specifically, the belt member 26F abuts on the top surface having a higher density of fiber, and does not abut on the back surface having a lower density of fiber. Accordingly, even if the adhesive of the double-sided tape 4j, which is located on the side closer to the back surface of the preceding material 3a, enters (permeates) into the preceding material 3a, the adhesive is prevented from penetrating through the preceding material 3a and being attached to the belt member 26F (so-called penetration); This is because a portion having high density of fiber on the top surface side blocks the adhesive. In addition, a portion where the belt member 26F abuts on (the top surface) is a portion having a high density of fiber (in other words, a portion whose fibers are compressed to high densities to be hard). Accordingly, compared to a portion having a low density of fiber (in other words, a portion whose fibers are not well compressed to be soft), even if the adhesive is attached to the belt member 26F, there is an advantages that the adhesive on the belt member 26F is less likely to be attached to the preceding material 3a.

When the subsequent material 3f is bonded with the preceding material 3a, the preceding material 3a and the subsequent material 3f become transported in an integrated manner by a transport section (the transport roll 29R and feeding rotation shafts 24 and 24), moving in the direction of transport the bonded portion 3j where the preceding material 3a and the subsequent material 3f are bonded. The controller controls the actuator 26G of the cutter mechanism 28 at a predetermined time, performing the cutting process of the preceding material 3a. Specifically, the cutter blade 28B of the cutter mechanism 28 abuts on the preceding material 3a, and thereby the preceding material 3a is cut.

In the present embodiment, as shown in FIG. 3, at a position upstream in the direction of transport from the bonded portion 3j where the preceding material 3a and the subsequent material 3f are bonded, the preceding material 3a is cut (at a position indicated by symbol A2 in FIG. 3). For only the purpose of splicing the subsequent material 3f with the preceding material 3a, the preceding material 3a may be cut at a position of the back end of the bonded portion 3j (at a position indicated by symbol A1 in FIG. 3). However, in the present embodiment, for another purpose (to be described later in detail), the preceding material 3a is cut at a farther back position. Accordingly, on the upstream side in the direction of transport from the bonded portion 3j, formed is a tail-shaped portion of the preceding material 3a projecting beyond the bonded portion 3j (a portion from A1 to A2 in FIG. 3; for convenience, hereinafter referred to as a fin-like portion 3at). In the present embodiment, for the foregoing other purpose, the timing of cutting is determined so that the length of the fin-like portion 3at is a predetermined length. As illustrated in FIG. 3, the timing of cutting is further later than the timing of bonding (after the bonded portion 3j has passed the entrance roll 41u). Thus, in the present embodiment, the cutter mechanism 28 cuts the preceding material 3a at a position upstream in the direction of transport from the bonded portion 3j where the preceding material 3a and the subsequent material 3f are bonded, and the fin-like portion 3at of the preceding material 3a is formed on the upstream side from the bonded portion 3j.

When the preceding material 3a has been cut, the controller decreases the rotation speed of the feeding rotation shaft 24 placed at the preceding-material coil position P3Ca, and stops the rotation of the rotation shaft 24. As a result of continuing the transport of the material 3 by the transport section, the fin-like portion 3at follows the bonded portion 3j and moves in the direction of transport. Then, the controller controls the actuator 26G of the pressing mechanism 26 at a predetermined timing, moving (back) the belt member 26F from the abutting position to the stand-by position. In the present embodiment, the cutter mechanism 28 cuts the preceding material 3a with the belt member 26F being located at the abutting position, and after cutting of the preceding material 3a is completed, the belt member 26F is moved back to the stand-by position. In other words, the belt member 26F does not move back to the stand-by position immediately after bonding the subsequent material 3f with the preceding material 3a, but the moving-back process is performed after the cutting process of the preceding material 3a.

In the present embodiment, as shown in FIG. 4, the belt member 26F is moved back from the abutting position to the stand-by position after releasing the abutting of the fin-like portion 3at of the material 3 being transporting on the belt member 26F. Specifically, the belt member 26F is moved to the stand-by position after the back end of the fin-like portion 3at (indicated by symbol A2) have passed the belt member 26F (in other words, the downstream end P1 of the abutting portion 3b of the material 3 which abuts on the belt member 26F).

After the moving process in which the belt member 26F moves to the stand-by position has been performed, the transport section (the transport roll 29R and the feeding rotation shaft 24) continues the transport of the material 3. At this stage, the material 3 is moving in the direction of transport with the following parts being lined in this order: the preceding material 3a (the preceding material alone); the bonded portion 3j; a stacking portion 3d where the fin-like portion 3at and the subsequent material 3f are stacked; and the subsequent material 3f (the subsequent material alone), as shown in FIG. 4. Note that the stacking portion 3d is a portion from A1 to A2 in FIG. 4 where the entire part of the fin-like portion 3at is stacked on the subsequent material 3f, and therefore the length of the stacking portion 3d is equal to the length of the fin-like portion 3at. That is, while the material 3 being wound around the rolls (the entrance roll 41u, the moving roll 41m, the exit roll 41d, the transport roll 29R, end the like), the transport section (the transport roll 29R and the feeding rotation shaft 24) continues the transport of the material 3. In the material 3, the bonded portion 3j and the stacking portion 3d are provided so as to be side-by-side along the direction of transport, and the bonded portion 3j and the stacking portion 3d moves in the direction of transport, sequentially passing the rolls.

After the controller stops the rotation of the feeding rotation shaft 24 that is located at the preceding-material coil position P3Ca, a worker then removes the paper tube 3p of the preceding material 3a from the feeding rotation shaft 24, and fits a new unfed material coil 3Cn onto the feeding rotation shaft 24. The double-sided tape 4j for bonding is provided in the leading end portion 3ne of the material 3n that is located on the outer circumferential surface 3Cns of the new material coil 3Cn, and double-sided tape 4k for provisional retaining is provided on the back surface of the leading end portion 3ne.

The controller controls the servomotor of the turret 22 such that the turret 22 pivots in the clockwise direction, when the controller determines that the turret 22 satisfies a pivotable condition; more specifically, the outer diameter of subsequent-material coil 3Cf that is located at the subsequent-material coil position P3Cf is reduced due to the feeding, and as a result the controller determines that the turret 22 can pivot without the coil 3Cf interfering with the floor portion LMB of the manufacturing line LM, the belt member 26F located at the stand-by position, the cutter blade 28B located at the stand-by position and the like.
Accordingly, the subsequent-material coil 3Cf moves downward along an arc-shaped path and then moves upward. Consequently, the material coil 3Cf moves to the preceding-material coil position P3Ca, and the new unfed material coil 3Cn moves to the subsequent-material coil position P3Cf. The controller then repeats the above-described bonding operation when the next bonding operation timing is reached.

As mentioned above, tension control for suppressing variation in tension is performed also during the bonding operation (material splicing process). More specifically, that tension control is always performed from before starting the material splicing process, through the material splicing process (a process for pressing against belt member), through the cutting process, through the moving-back process for moving back the belt member, to after the moving-back process. In the present embodiment, the controller adjusts the rotation speeds of the feeding rotation shafts 24 and 24 of the transport section, controlling the transport of the material 3 so that the moving roll 41m is positioned at the reference position (so that the size of the loop 3L is constant). Accordingly, even if a temporary variation in tension occurs due to various reasons (for convenience, referred to as an abnormal state), that controlling immediately recovers normal tension (for convenience, referred to as a normal state).

### = Length of Fin-like portion 3at =

As mentioned above, in the present embodiment, when bonding the preceding material 3a and the subsequent material 3f, in the cutting process of the preceding material 3a, the preceding material 3a is cut so that the fin-like portion 3at is formed next to the bonded portion 3j. The cutting is performed so that the length of the fin-like portion 3at is a predetermined length.

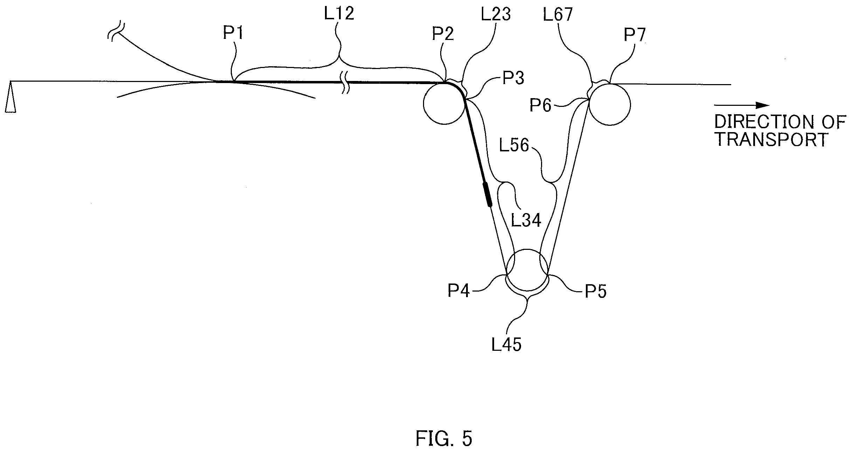

Here, with reference to FIGS. 5 to 11, in forming the fin-like portion, how the length of the fin-like portion 3at (in other words, the length of the stacking portion 3d) is determined 3at will be described below. What is the advantages when the length of the fin-like portion 3at (the stacking portion 3d) is determined in such a manner will be described below.

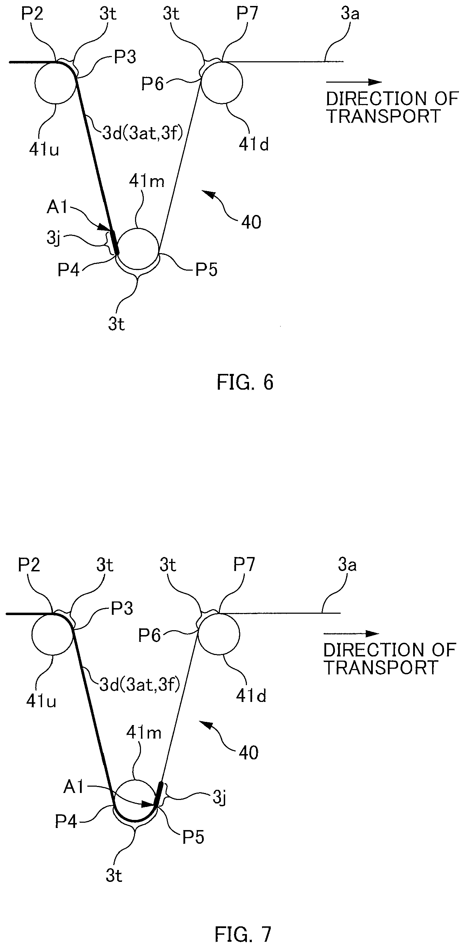

FIG. 5 is a diagram corresponding to FIG. 3, and shows Positions P1 to P7 of the material 3 in the transport path and the path lengths of the material 3 between the Positions (a length of a transport path) L12 to L67. FIGS. 6 to 11 are diagrams corresponding to FIG. 3, and are diagrams illustrating advantages (effects) of the present embodiment. In FIGS. 5 to 11, the moving roll 41m is positioned at the reference position.

In the present embodiment, the total length of the bonded portion 3j and the stacking portion 3d (the fin-like portion 3at) is larger than the path length (L34) of the material 3 from the downstream end P3 of a winding portion 3t of the material 3 which is wound around the entrance roll 41u to the upstream end P4 of a winding portion 3t of the material 3 which is wound around the moving roll 41m positioned at the reference position. That is, the cutter mechanism 28 (the cutter blade 28B) cuts the preceding material 3a so that the total length of the bonded portion 3j and the stacking portion 3d (the fin-like portion 3at) is larger than the path length (L34) of the material 3 from the downstream end P3 to the upstream end P4. This realizes the following advantages.

As shown in FIG. 6, when the material 3 is transported and the bonded portion 3j reaches the moving roll 41m, the moving roll 41m suddenly comes into contact with a portion having a different rigidity. Specifically, during when the preceding material 3a (the preceding material alone) passes through the moving roll 41m, a portion in which the preceding material 3a, the subsequent material 3f and the double-sided tape 4j are joined (a portion having a higher rigidity) suddenly passes the moving roll 41m. In such a state, the material 3 is impacted on, and there is a possibility that fluttering (rampage) of the material 3 occurs. The fluttering (rampage) leads to occurrence of variation in tension.

As opposed thereto, in the present embodiment, since as shown in FIG. 6 the total length of the bonded portion 3j and the stacking portion 3d (the fin-like portion 3at) is larger than the path length (L34) from the downstream end P3 to the upstream end P4, the stacking portion 3d is reliably wound around the entrance roll 41u under such a condition, making a portion from the upstream end P4 to the downstream end P3 be a double portion of the material 3 (the bonded portion 3j or the stacking portion 3d, that is a portion including more than one material). Accordingly, even if the material 3 is impacted on under the abovementioned condition, high rigidity of the double portion can suppress fluttering (rampage) of the material 3, making it possible to suppress variation in tension in the material 3.

In the present embodiment, the length of the stacking portion 3d (the fin-like portion 3at) is larger than the path length (L34 + L45) of the material 3 from the downstream end P3 of the winding portion 3t of the material 3 which is wound around the entrance roll 41u to the downstream end P5 of the winding portion 3t of the material 3 which is wound around the moving roll 41m positioned at the reference position. That is, the cutter mechanism 28 (the cutter blade 28B) cuts the preceding material 3a so that the length of the stacking portion 3d (the fin-like portion 3at) is larger than the path length (L34 + L45) of the material 3 from the downstream end P3 to the downstream end P5.

Accordingly, at the time when the bonded portion 3j has reached the moving roll 41m, and from the time of the reach (see FIG. 6) until the bonded portion 3j passes through out of the moving roll 41m (see FIG. 7), the stacking portion 3d is reliably wound around the entrance roll 41u, making a portion from the upstream end P4 to the downstream end P3 be a double portion of the material 3 (the bonded portion 3j or the stacking portion 3d, that is a portion including more than one material) (see FIGS. 6 and 7). Accordingly, it is possible to more appropriately suppress fluttering (rampage) of the material 3, making it possible to more appropriately suppress variation in tension in the material 3.

In the present embodiment, the total length of the bonded portion 3j and the stacking portion 3d (the fin-like portion 3at) is larger than the path length (L34 + L45 + L56) of the material 3, from the downstream end P3 of the winding portion 3t of the material 3 which is wound around the entrance roll 41u, through the moving roll 41m positioned at the reference position, to the upstream end P6 of the winding portion 3t of the material 3 which is wound around the exit roll 41d. That is, the cutter mechanism 28 (the cutter blade 28B) cuts the preceding material 3a so that the total length of the bonded portion 3j and the stacking portion 3d (the fin-like portion 3at) is larger than the path length (L34 + L45 + L56) of the material 3 from the downstream end P3 to the upstream end P6.

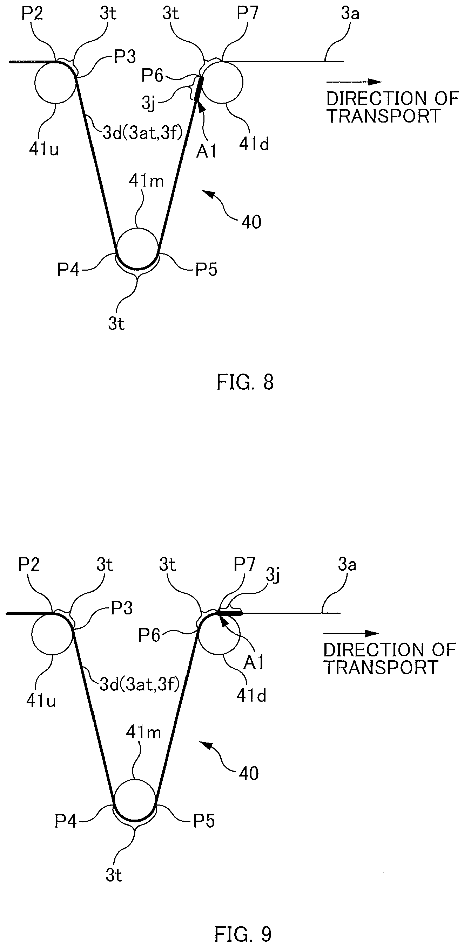

Accordingly, as shown in FIG. 8, when the bonded portion 3j reaches the exit roll 41d, the stacking portion 3d is reliably wound around the entrance roll 41u, making a portion from the upstream end P6 to the downstream end P3 (that is, the entire loop 3L of the accumulating device 40) be a double portion of the material 3 (the bonded portion 3j or the stacking portion 3d, that is a portion including more than one material) . Accordingly, it is possible to suppress not only fluttering (rampage) of the material 3 due to impact by contact of the bonded portion 3j with the moving roll 41m (hereinafter referred to as impact related to the moving roll 41m), but also fluttering (rampage) of the material 3 due to impact by contact of the bonded portion 3j with the exit roll 41d (hereinafter referred to as impact related to the exit roll 41d). Accordingly, it is possible to more appropriately suppress fluttering (rampage) of the material 3, making it possible to more appropriately suppress variation in tension in the material 3.

Meanwhile, in the case where the length of the fin-like portion 3at is set as mentioned above, there are advantages of not only suppressing fluttering (rampage) of the material 3 due to impact related to the moving roll 41m, but also suppressing fluttering (rampage) of the material 3 due to impact related to the exit roll 41d. However, the length of the fin-like portion 3at becomes remarkably long.

Here, assuming that adverse effect by such a phenomenon is mainly considered (e.g., if the length of the fin-like portion 3at is too long, the fin-like portion 3at is easy to be cut out of the material 3; the fin-like portion 3at that has been cut out has the potential to adversely affect the devices). In this case, there is an alternative of cutting the preceding material 3a in a manner to satisfy the following conditions: the length of the stacking portion 3d (the fin-like portion 3at) is larger than the path length (L34 + L45) of the material 3 from the downstream end P3 to the downstream end P5; and the total length of the bonded portion 3j and the stacking portion 3d (the fin-like portion 3at) is smaller than the path length (L34 + L45 + L56) of the material 3 from the downstream end P3 to the upstream end P6.

That is, the former one should preferably be selected if focusing on suppressing fluttering (rampage) of the material 3 and variation in tension in the material 3. And, the latter one should preferably be selected if adverse effect by elongation of the fin-like portion 3at would like to be suppressed concurrently suppressing fluttering (rampage) of the material 3 and variation in tension in the material 3.

Note that, in the case of the former one, the length of the fin-like portion 3at may be further elongated as follow. Specifically, the length of the stacking portion 3d (the fin-like portion 3at) is made larger than the path length (L34 + L45 + L56 + L67) of the material 3, from the downstream end P3 of the winding portion 3t of the material 3 which is wound around the entrance roll 41u, through the moving roll 41m positioned at the reference position, to the downstream end P7 of the winding portion 3t of the material 3 which is wound around the exit roll 41d. That is, the cutter mechanism 28 (the cutter blade 28B) cuts the preceding material 3a so that the length of the stacking portion 3d (the fin-like portion 3at) is larger than the path length (L34 + L45 + L56 + L67) of the material 3 from the downstream end P3 to the downstream end P7.

Accordingly, at the time when the bonded portion 3j has reached the exit roll 41d, and from the time of the reach (see FIG. 8) until the bonded portion 3j passes through out of the exit roll 41d (see FIG. 9), the stacking portion 3d is reliably wound around the entrance roll 41u, making a portion from the upstream end P6 to the downstream end P3 (that is, the entire loop 3L of the accumulating device 40) be a double portion of the material 3 (the bonded portion 3j or the stacking portion 3d, that is a portion including more than one material) (see FIGS. 8 and 9). Accordingly, it is possible to more appropriately suppress fluttering (rampage) of the material 3, making it possible to more appropriately suppress variation in tension in the material 3.

In the present embodiment, the total length of the bonded portion 3j and the stacking portion 3d (the fin-like portion 3at) is larger than the path length (L23 + L34) of the material 3 from the upstream end P2 of the winding portion 3t of the material 3 which is wound around the entrance roll 41u to the upstream end P4 of the winding portion 3t of the material 3 which is wound around the moving roll 41m positioned at the reference position. That is, the cutter mechanism 28 (the cutter blade 28B) cuts the preceding material 3a so that the total length of the bonded portion 3j and the stacking portion 3d (the fin-like portion 3at) is larger than the path length (L23 + L34) of the material 3 from the upstream end P2 to the upstream end P4.

Accordingly, as shown in FIG. 6, when the bonded portion 3j reaches the moving roll 41m, the stacking portion 3d is reliably wound around the entirety of a portion of the entrance roll 41u with which the material 3 can come into contact, not a part of the entrance roll 41u. That is, the portion from the upstream end P4 to the upstream end P2 becomes a double portion of the material 3 (the bonded portion 3j or the stacking portion 3d, that is a portion including more than one material). Accordingly, even if the material 3 is impacted on under the abovementioned condition, high rigidity of the double portion that is reliably wound around the entrance roll 41u can more appropriately suppress fluttering (rampage) of the material 3, making it possible to more appropriately suppress variation in tension in the material 3.

Further, in the present embodiment, the total length of the bonded portion 3j and the stacking portion 3d (the fin-like portion 3at) is larger than the path length (L12 + L23 + L34) of the material 3 from the downstream end P1 of the abutting portion 3b of the material 3 which abuts on the belt member 26F to the upstream end P4 of the winding portion 3t of the material 3 which is wound around the moving roll 41m positioned at the reference position. That is, the cutter mechanism 28 (the cutter blade 28B) cuts the preceding material 3a so that the total length of the bonded portion 3j and the stacking portion 3d (the fin-like portion 3at) is larger than the path length (L12 + L23 + L34) of the material 3 from the downstream end P1 to the upstream end P4. After the bonded portion 3j of the material 3 being transported reaches the moving roll 41m, the belt member 26F is moved back from the abutting position to the stand-by position.

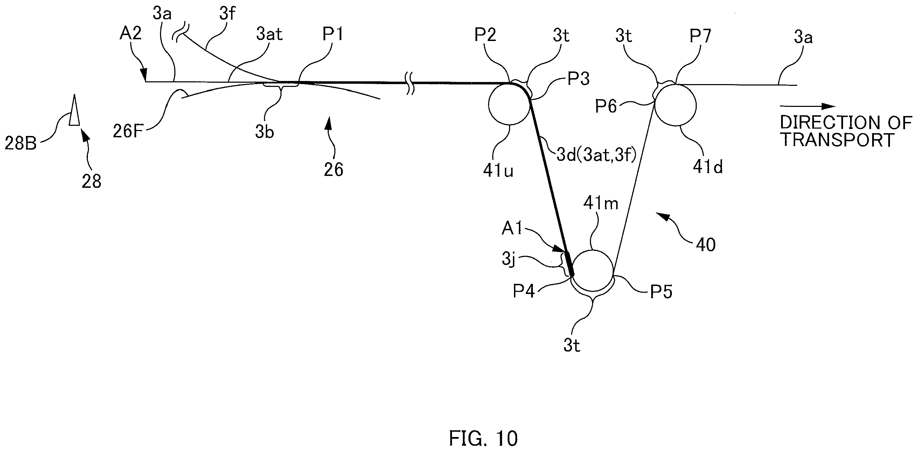

Accordingly, as shown in FIG. 10, when the bonded portion 3j reaches the moving roll 41m, the stacking portion 3d is reliably wound around not only the entrance roll 41u but also the belt member 26F, making a portion from the upstream end P4 to the downstream end P1 be a double portion of the material 3 (the bonded portion 3j or the stacking portion 3d, that is a portion including more than one material). Accordingly, even if the material 3 is impacted on under the abovementioned condition, high rigidity of the double portion wound around the entrance roll 41u and the belt member 26F can more appropriately suppress fluttering (rampage) of the material 3, making it possible to more appropriately suppress variation in tension in the material 3.

Meanwhile, in the case where the length of the fin-like portion 3at is set as mentioned above, there is an advantage that high rigidity of the double portion wound around the entrance roll 41u and the belt member 26F suppresses more appropriately fluttering (rampage) of the material 3. However, the length of the fin-like portion 3at becomes remarkably long.

Accordingly, if adverse effect by such a phenomenon is mainly considered, there is an alternative of cutting the preceding material 3a in a manner to satisfy the following conditions: the total length of the bonded portion 3j and the stacking portion 3d (the fin-like portion 3at) is larger than the path length (L23 + L34) of the material 3 from the upstream end P2 to the upstream end P4; and the total length of the bonded portion 3j and the stacking portion 3d (the fin-like portion 3at) is smaller than the path length (L12 + L23 + L34) of the material 3 from the downstream end P1 to the upstream end P4.

That is, the former one should preferably be selected if focusing on suppressing fluttering (rampage) of the material 3 and variation in tension in the material 3. And, the latter one should preferably be selected if adverse effect by elongation of the fin-like portion 3at would like to be suppressed concurrently suppressing fluttering (rampage) of the material 3 and variation in tension in the material 3.

Note that, in the case of the former one, the length of the fin-like portion 3at may be further elongated as follow. Specifically, the length of the stacking portion 3d (the fin-like portion 3at) is made larger than the path length (L12 + L23 + L34 + L45) of the material 3, from the downstream end P1 of the abutting portion 3b of the material 3 which abuts on the belt member 26F to the downstream end P5 of the winding portion 3t of the material 3 which is wound around the moving roll 41m positioned at the reference position. That is, the cutter mechanism 28 (cutter blade 28B) cuts the preceding material 3a so that the length of the stacking portion 3d (the fin-like portion 3at) is larger than the path length (L12 + L23 + L34 + L45) of the material 3 from the downstream end P1 to the downstream end P5. In addition, after the bonded portion 3j of the material 3 being transported has passed the moving roll 41m, the belt member 26F is moved back from the abutting position to the stand-by position.

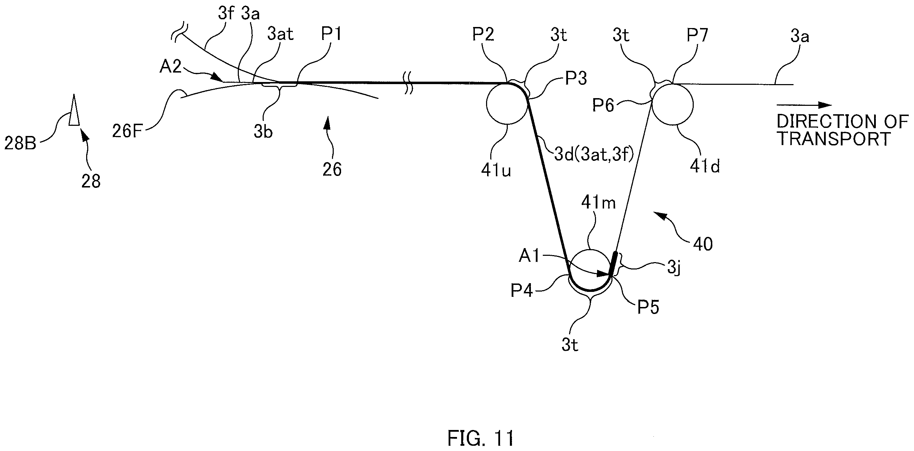

Accordingly, at the time when the bonded portion 3j has reached the moving roll 41m, and from the time of the reach (see FIG. 10) until the bonded portion 3j passes through out of the moving roll 41m (see FIG. 11), the stacking portion 3d is reliably wound around the entrance roll 41u and the belt member 26F, making a portion from the upstream end P4 to the downstream end P1 be a double portion of the material 3 (the bonded portion 3j or the stacking portion 3d, that is a portion including more than one material) (see FIGS. 10 and 11). Accordingly, it is possible to more appropriately suppress fluttering (rampage) of the material 3, making it possible to more appropriately suppress variation in tension in the material 3.

In order for the length of the stacking portion 3d (the fin-like portion 3at) to be equal to a predetermined length, various methods may be employed as a method for determining the timing of cutting. There is an example as follow. The rotation speed of the material coil 3C (or the feeding rotation shafts 24 and 24) is always monitored by a rotary encoder or the like, and the (decreasing) outer diameter of the material coil 3C is always monitored by a laser displacement meter, an ultrasonic displacement meter or the like. From these monitored values (which can vary over time), the feeding speed of material 3 can be obtained. And, it is sufficient that the material 3 cuts at a timing when the integral of the feeding speed over the elapsed time after the bonding (after forming the bonded portion 3j) becomes equal to the predetermined length (a desired length).

As for other methods, there is a method, for example, in which the material 3 cuts at a timing when the bonded portion 3j is detected by a sensor (e.g., a CCD camera) or at a timing when a predetermined time has passed after the detecting. Here, such a sensor can detect the bonded portion 3j and is installed at a certain position on the transport path.

In any way, it is sufficient that the cutting is performed so that the total length of the bonded portion 3j and the stacking portion 3d (the fin-like portion 3at) is larger (smaller) than the lengths between the Positions P1 to P7 or so that the length of the stacking portion 3d (the fin-like portion 3at) is larger (smaller) than the lengths between the Positions P1 to P7; this is because it is not necessary to perform cutting so as to be precisely equal to the lengths between the Positions P1 to P7. Accordingly, the timing of cutting may be determined with margins.

In order to appropriately suppress variation in tension in the material 3as in the abnormal state as well as in the normal state (that is, the moving roll 41m positioned at the reference position), it is sufficient to determined the timing of cutting as follow: leaving a margin corresponding to the length of difference between when the size of the loop 3L is maximum and when the moving roll 41m is positioned at the reference position so that the abovementioned conditions are satisfied not only when the moving roll 41m is positioned at the reference position but also the moving roll 41m is positioned at a position where the size of the loop 3L is maximum.

### = Other Embodiments =

The above embodiment of the invention is for facilitating understanding of the invention, and are not limiting of the invention. The invention can of course be altered and improved without departing from the gist thereof, and equivalents are intended to be embraced therein.

In the foregoing embodiment, an absorbent article is exemplified by a so-called tape-type disposable diaper 1. However, the invention is not limited thereto. For example, a pull-one disposable diaper may be employed. In addition, the absorbent article is not limited to the disposable diaper 1. That is, it may be any article that absorbs excreted fluid from the wearer. For example, the absorbent article may be a sanitary napkin, a urine absorbing pad, or the like.

In the foregoing embodiment, the material 3 is exemplified by the continuous sheet 3 which is a fiber assembly. However, the invention is not limited thereto. For example, the material 3 may be a film.

In the foregoing embodiment, the subsequent material 3f is bonded to the preceding material 3a with the adhesive member. However, the invention is not limited thereto. For example, the bonding may be made by welding such as heat sealing, ultrasonic sealing, or may be made by any other method for bonding. The adhesive member is exemplified by the double-sided tape 4j on which adhesive is provided. However, the invention is not limited thereto. The adhesive member itself may be adhesive such as glue.

In the foregoing embodiment, the cutter mechanism 28 cuts the preceding material 3a with the belt member 26F being located at the abutting position. In other words, the belt member 26F does not move back to the stand-by position immediately after bonding the subsequent material 3f with the preceding material 3a, but the moving-back process is performed after the cutting process of the preceding material 3a. However, the invention is not limited thereto. The belt member 26F may have already moved back to the stand-by position when the cutter mechanism 28 cuts the preceding material 3a.

However, the belt member 26F reliably abuts on the preceding material 3a when cutting the preceding material 3a, and thereby a portion from the downstream end P1 of the abutting portion 3b of the material 3 which abuts on the belt member 26F to the upstream end P2 of the winding portion 3t of the material 3 which is wound around the entrance roll 41u can be a double portion. Accordingly, even if the material 3 is impacted on during cutting, high rigidity of the double portion can more appropriately suppress fluttering (rampage) of the material 3, making it possible to more appropriately suppress variation in tension in the material 3. In this point, the foregoing embodiment is more preferable.

In the foregoing embodiment, the belt member 26F is moved back from the abutting position to the stand-by position after releasing the abutting of the fin-like portion 3at of the material 3 being transporting on the belt member 26F. That is, the belt member 26F is moved to the stand-by position after the back end of the fin-like portion 3at (indicated by symbol A2 in FIG. 4) have passed the belt member 26F (in other words, the downstream end P1 of the abutting portion 3b of the material 3 which abuts on the belt member 26F). However, the invention is not limited thereto. The belt member 26F may be moved back from the abutting position to the stand-by position before releasing the abutting of the fin-like portion 3at of the material 3 being transported on the belt member 26F (while the fin-like portion 3at abutting on the belt member 26F).

However, a portion from the downstream end P1 to the upstream end P2 can be a double portion over a period as long as possible till the fin-like portion 3at has reached (passed) the downstream end P1. High rigidity of the double portion can suppress variation in tension in the material 3 caused by disturbance, more appropriately (during more longer period). In this point, the foregoing embodiment is more preferable.

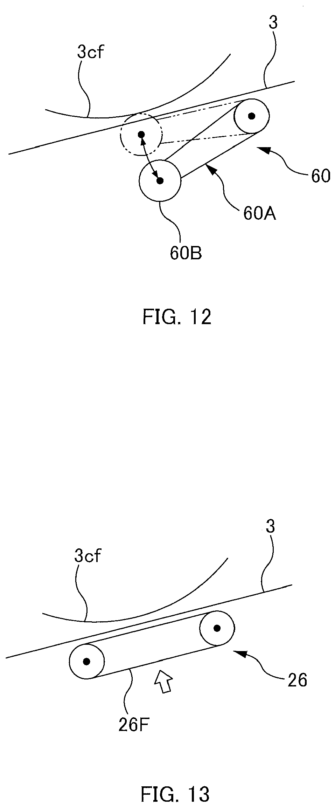

In the foregoing embodiment, the abutting member is exemplified by the belt member 26F. However, the invention is not limited thereto. For example, the abutting member may be the press roll 60B. That is, the following configuration may be employed: as shown in FIG. 12, the pressing mechanism 60 includes the swinging arm 60A and the press roll 60B provided on a swinging end of the swinging arm in a manner of being capable of rotating, and the press roll 60B abuts on the material 3 (the preceding material 3a).

In the case where the press roll 60B serves as the abutting member, an contact area which the press roll 60B is in contact with the material 3 is small (substantially point-to-point contact), making the material 3 easier to be depressed. The depression of the material 3 changes the transport path of the material 3 by the extent corresponding to the depression (the path length changes), causing variation in tension in the material 3 due to the path change.

As opposed thereto, in the case where the belt member 26F serves as the abutting member, compared to the case of the press roll 60B, an contact area which the press roll 60B is in contact with the material 3 is large (surface-to-surface contact as shown in the abutting portion 3b in FIG. 3), and the depression of the material 3 is small. This makes it possible to more appropriately suppress variation in tension in the material 3. In this point, the foregoing embodiment is more preferable.

In the foregoing embodiment, the belt member 26F rotates due to rotations of the first rotation roller 26B and the second rotation roller 26E while the belt member 26F being wound around the first rotation roller 26B and the second rotation roller 26E. And, the belt member 26F moves from the stand-by position to the abutting position by swinging about a swing axis, which is the central axis of the first rotation roller 26B (that is, the first fixed shaft 26A). However, the invention is not limited to such abutting by swinging operation. As shown in FIG. 13, the belt member 26F may move (abuts on) from the stand-by position to the abutting position in a straight movement operation.

In the case where the belt member 26F moves from the stand-by position to the abutting position in the foregoing swinging operation (or in the case of moving back from the abutting position to the stand-by position), the extent of path change of the material 3 in moving (in moving back) is moderate (the ratio of change of the path length is small) compared to the cases in the straight movement operation showing in FIG. 13. That is, in the case of swinging operation, the path changes gradually (not suddenly) compared to the straight movement operation, it is possible to avoid rapid variation in tension in the material 3. This makes it possible to more appropriately suppress variation in tension in the material 3. In this point, the foregoing embodiment is more preferable.

### [Reference Signs List]

1 disposable diaper(absorbent article), 2 absorbent body,
3 continuous sheet(material),
3C material coil, 3Ca preceding-material coil, 3Cf subsequent-material coil,
3Cfs outer circumferential surface, 3Cn material coil, 3Cns outer circumferential surface,
3L loop,
3a preceding material, 3at fin-like portion, 3b abutting portion, 3d stacking portion, 3f subsequent material, 3fe leading end portion, 3j bonded portion, 3n material, 3ne leading end portion,
3p paper tube, 3s leak-proof sheet, 3t winding portion
4j double-sided tape(adhesive member), 4k double-sided tape,
10 material supplying device, 20 material-splicing device, 21 support plate, 22 turret,
24 feeding rotation shaft (rotation shaft),
26 pressing mechanism, 26A first fixed shaft (central axis), 26B first rotation roller, 26C swinging arm, 26D second fixed shaft, 26E second rotation roller, 26F belt member (abutting member), 26G actuator,
28 cutter mechanism, 28A arm member, 28B cutter blade,
28C actuator,
29R transport roll,
40 accumulating device (dancer unit), 41A arm member, 41C actuator,
41d exit roll, 41m moving roll, 41u entrance roll,
50 turn bar (direction-of-transport changing member),
60 pressing mechanism, 60A swinging arm 60B press roll (abutting member),
110 processing unit (processing device), 110a depositing device, 110b cutting device,
110c pressing device, 110d leg-opening cutting device,
110e end-cutting device,
LM manufacturing line, LMB floor portion,
P3Ca preceding-material coil position, P3Cf subsequent-material coil position,
C22 pivot axis, C28A rotation axis,
C41A rotation axis, C41u rotation axis, C41m rotation axis, C41d rotation axis

## Claims

1. A method for splicing a material associated with an absorbent article, comprising:
transporting the material in a direction of transport while the material being wound around an entrance roll (41u) and a moving roll (41m),
the entrance roll (41u) and the moving roll (41m) included in a dancer unit (40);
splicing a subsequent material (3f) with a preceding material (3a) by bonding a leading end portion of the subsequent material (3f) with the preceding material (3a),
the preceding material (3a) being the material that is transported;
forming a fin-like portion (3at) of the preceding material (3a) upstream in the direction of transport from a bonded portion (3j) where the preceding material (3a) and the subsequent material (3f) are bonded,
the forming being performed by cutting the preceding material (3a) at a position upstream from the bonded portion (3j);
continuing transport of the material in which the bonded portion (3j) and a stacking portion (3d) are provided side-by-side along the direction of transport, while the material being wound around the entrance roll (41u) and the moving roll (41m),
the stacking portion (3d) being a portion where the fin-like portion (3at) and the subsequent material (3f) are stacked; and
controlling transport of the material so that the moving roll (41m) is located at a reference position,
in the forming the fin-like portion (3at),
the preceding material (3a) is cut so that a total length of the bonded portion (3j) and the stacking portion (3d) is larger than a path length of the material from a downstream end (P3) of a winding portion (3t) of the material to an upstream end (P4) of another winding portion of the material,
the winding portion (3t) being a portion wound around the entrance roll (41u),
the other winding portion (3t) being a portion wound around the moving roll (41m) located at the reference position.

2. A method for splicing a material according to claim 1, wherein
in the forming the fin-like portion (3at),
the preceding material (3a) is cut so that a length of the stacking portion (3d) is larger than a path length of the material from the downstream end of the winding portion (3t) of the material to a downstream end of the other winding portion (3t) of the material,
the winding portion (3t) being a portion wound around the entrance roll (41u),
the other winding portion (3t) being a portion wound around the moving roll (41m) located at the reference position.

3. A method for splicing a material according to claim 2, wherein
the material is transported in the direction of transport while the material being wound around the entrance roll (41u), the moving roll (41m), and an exit roll (41d) of the dancer unit (40),
after splicing the subsequent material (3f) and the preceding material (3a),
transport of the material in which the bonded portion (3j) and the stacking portion (3d) are provided side-by-side along the direction of transport continues while the material being wound around the entrance roll (41u), the moving roll (41m), and the exit roll (41d), and
in the forming the fin-like portion (3at),
the preceding material (3a) is cut so that the total length of the bonded portion (3j) and the stacking portion (3d) is larger than a path length of the material from the downstream end of the winding portion (3t) of the material through the moving roll (41m) located at the reference position, to an upstream end of another winding portion (3t) of the material,
the winding portion (3t) being a portion wound around the entrance roll (41u),
the other winding portion (3t) being a portion wound around the exit roll (41d).

4. A method for splicing a material according to claim 3, wherein
in the forming the fin-like portion (3at),
the preceding material (3a) is cut so that the length of the stacking portion (3d) is larger than a path length of the material from the downstream end of the winding portion (3t) of the material, through the moving roll (41m) located at the reference position, to a downstream end of the other winding portion (3t) of the material,
the winding portion (3t) being a portion wound around the entrance roll (41u),
the other winding portion (3t) being a portion wound around the exit roll (41d).

5. A method for splicing a material according to claim 2, wherein
the material is transported in the direction of transport while the material being wound around the entrance roll (41u), the moving roll (41m), and an exit roll (41d) of the dancer unit (40),
after splicing the subsequent material (3f) and the preceding material (3a),
transport of the material in which the bonded portion (3j) and the stacking portion (3d) are provided side-by-side along the direction of transport continues while the material being wound around the entrance roll (41 u), the moving roll (41m), and the exit roll (41d), and
in the forming the fin-like portion (3at),
the preceding material (3a) is cut so that the total length of the bonded portion (3j) and the stacking portion (3d) is smaller than a path length of the material from the downstream end of the winding portion (3t) of the material, through the moving roll (41m) located at the reference position, to an upstream end of another winding portion (3t) of the material,
the winding portion (3t) being a portion wound around the entrance roll (41 u),
the other winding portion (3t) being a portion wound around the exit roll (41d).

6. A method for splicing a material according to any one of claims 1 to 5, wherein
in the forming the fin-like portion (3at),
the preceding material (3a) is cut so that the total length of the bonded portion (3j) and the stacking portion (3d) is larger than a path length of the material from an upstream end of the winding portion (3t) of the material to the upstream end of the other winding portion (3t) of the material,
the winding portion (3t) being a portion wound around the entrance roll (41u),
the other winding portion (3t) being a portion wound around the moving roll (41m) located at the reference position.

7. A method for splicing a material according to claim 6, wherein
when bonding the subsequent material (3f) with the preceding material (3a) being transported,
the preceding material (3a) is pressed against the subsequent material (3f) by moving an abutting member from a stand-by position to an abutting position where the abutting member abuts on the preceding material (3a),
after the bonded portion (3j) of the material being transported has reached the moving roll (41m),
the abutting member is moved back from the abutting position to the stand-by position, and
in the forming the fin-like portion (3at),
the preceding material (3a) is cut so that the total length of the bonded portion (3j) and the stacking portion (3d) is larger than a path length of the material from a downstream end of an abutting portion of the material to the upstream end of the other winding portion (3t) of the material,
the abutting portion being a portion that abuts on the abutting member,
the other winding portion (3t) being a portion wound around the moving roll (41m) located at the reference position.

8. A method for splicing a material according to claim 7, wherein
after the bonded portion (3j) of the material being transported has passed the moving roll (41m),
the abutting member is moved back from the abutting position to the stand-by position, and
in the forming the fin-like portion (3at),
the preceding material (3a) is cut so that a length of the stacking portion (3d) is larger than a path length of the material from the downstream end of the abutting portion of the material to the upstream end of the other winding portion (3t) of the material to a downstream end of the other winding portion (3t) of the material,
the abutting portion being a portion that abuts on the abutting member,
the other winding portion (3t) being a portion wound around the moving roll (41m) located at the reference position.

9. A method for splicing a material according to claim 6, wherein
when bonding the subsequent material (3f) with the preceding material (3a) being transported,
the preceding material (3a) is pressed against the subsequent material (3f) by moving an abutting member from a stand-by position to an abutting position where the abutting member abuts on the preceding material (3a), and
in the forming the fin-like portion (3at),
the preceding material (3a) is cut so that the total length of the bonded portion (3j) and the stacking portion (3d) is smaller than a path length of the material from a downstream end of an abutting portion of the material to the upstream end of the other winding portion (3t) of the material,
the abutting portion being a portion that abuts on the abutting member,
the other winding portion (3t) being a portion wound around the moving roll (41m) located at the reference position.

10. A method for splicing a material according to any one of claims 1 to 9, wherein
when bonding the subsequent material (3f) with the preceding material (3a) being transported,
the preceding material (3a) is pressed against the subsequent material (3f) by moving an abutting member from a stand-by position to an abutting position where the abutting member abuts on the preceding material (3a), and
the preceding material (3a) is cut with the abutting member being located at the abutting position.

11. A method for splicing a material according to claim 10, wherein
after releasing the abutting of the fin-like portion (3at) of the material being transported on the abutting member,
the abutting member is moved back from the abutting position to the stand-by position.

12. A method for splicing a material according to any one of claims 1 to 11, wherein
when bonding the subsequent material (3f) with the preceding material (3a) being transported,
the preceding material (3a) is pressed against the subsequent material (3f) by moving an abutting member from a stand-by position to an abutting position where the abutting member abuts on the preceding material (3a), and
the abutting member is a belt member being capable of rotating.

13. A method for splicing a material according to claim 12, wherein
the belt member is a member
that rotates due to rotatings of a first rotation roller and a second rotation roller while the belt member being wound around the first rotation roller and the second rotation roller, and
that moves from the stand-by position to the abutting position by swinging about a swing axis,
the swing axis being a central axis of the first rotation roller.

14. A method for splicing a material according to any one of claims 1 to 13, wherein
the material is a continuous sheet being a fiber assembly,
one surface of the continuous sheet has a density of fiber higher than another surface of the continuous sheet, and
when bonding the subsequent material (3f) with the preceding material (3a) being transported,
the preceding material (3a) is pressed against the subsequent material (3f) while an adhesive member being sandwiched between the preceding material (3a) and the subsequent material (3f) by moving an abutting member from a stand-by position to an abutting position where the abutting member abuts on the one surface of the preceding material (3a).

15. A method for splicing a material according to any one of claims 1 to 14, wherein
the material is a continuous sheet being a fiber assembly,
one surface of the continuous sheet has a density of fiber higher than another surface of the continuous sheet, and
in the forming the fin-like portion (3at) by cutting the preceding material (3a),
a cutter blade enters from the one surface.

16. A device for supplying a material associated with an absorbent article, comprising:
a dancer unit (40) including an entrance roll (41u) and a moving roll (41m);
a transport section configured to transport the material in a direction of transport while the material wound around the entrance roll (41u) and the moving roll (41m);
a material splicing section configured to splice a subsequent material (3f) with a preceding material (3a) by bonding a leading end portion of the subsequent material (3f) with the preceding material (3a),
the preceding material (3a) being the material that is transported;
a cutting section that is configured to form a fin-like portion (3at) of the preceding material (3a) upstream in the direction of transport from a bonded portion (3j) where the preceding material (3a) and the subsequent material (3f) are bonded, whereby the forming being performed by cutting the preceding material (3a) at a position upstream from the bonded portion (3j), whereby the transport of the material in which the bonded portion (3j) and a stacking portion (3d) are provided side-by-side along the direction of transport being continued by the transport section while the material being wound around the entrance roll (41u) and the moving roll (41m),
the stacking portion (3d) being a portion where the fin-like portion (3at) and the subsequent material (3f) are stacked; and
a control section configured to control transport of the material so that the moving roll (41m) is located at a reference position,
the cutting section being configured to cut the preceding material (3a) so that a total length of the bonded portion (3j) and the stacking portion (3d) is larger than a path length of the material from a downstream end of a winding portion of the material to an upstream end of another winding portion (3t) of the material, whereby
the winding portion being a portion wound around the entrance roll (41u), and
the other winding portion (3t) being a portion wound around the moving roll (41m) located at the reference position.

## Patentansprüche

1. Verfahren zum Verbinden eines Materials, das einem absorbierenden Artikel zugeordnet ist, umfassend:
Fördern des Materials in einer Förderrichtung, während das Material um eine Eintrittsrolle (41 u) und eine bewegliche Rolle (41m) gewickelt wird,
wobei die Eintrittsrolle (41u) und die bewegliche Rolle (41m) in einer Tänzereinheit (40) umfasst sind;
Verbinden eines nachfolgenden Materials (3f) mit einem vorhergehenden Material (3a) durch Verbinden eines vorderen Endabschnitts des nachfolgenden Materials (3f) mit dem vorhergehenden Material (3a),
wobei das vorhergehende Material (3a) das Material ist, das gefördert wird;
Ausbilden eines rippenartigen Abschnitts (3at) des vorhergehenden Materials (3a) stromaufwärts in Förderrichtung aus einem verbundenen Abschnitt (3j) an dem das vorhergehende Material (3a) und das nachfolgende Material (3f) verbunden sind,
wobei das Ausbilden durch Schneiden des vorhergehenden Materials (3a) an einer Position stromaufwärts des verbundenen Abschnitts (3j) durchgeführt wird;
Weiterfördern des Materials, bei dem der verbundene Abschnitt (3j) und ein Stapelabschnitt (3d) entlang der Förderrichtung nebeneinander vorgesehen sind, während das Material um die Eintrittsrolle (41u) und die bewegliche Rolle (41m) gewickelt wird,
wobei der Stapelabschnitt (3d) ein Abschnitt ist, in dem der rippenartige Abschnitt (3at) und das nachfolgende Material (3f) gestapelt sind; und
Steuern der Förderung des Materials, so dass die bewegliche Rolle (41 m) an einer Referenzposition angeordnet ist,
wobei, beim Ausbilden des rippenartigen Abschnitts (3at),
das vorhergehende Material (3a) derart geschnitten ist, dass eine Gesamtlänge des verbundenen Abschnitts (3j) und des Stapelabschnitts (3d) größer ist als eine Weglänge des Materials von einem stromabwärtigen Ende (P3) eines Wicklungsabschnitts (3t) des Materials zu einem stromaufwärtigen Ende (P4) eines anderen Wicklungsabschnitts des Materials,
wobei der Wicklungsabschnitt (3t) ein Abschnitt ist, der um die Eintrittsrolle (41u) gewickelt ist,
wobei der andere Wicklungsabschnitt (3t) ein Abschnitt ist, der um die bewegliche Rolle (41m) gewickelt ist, die an der Referenzposition angeordnet ist.

2. Verfahren zum Verbinden eines Materials gemäß Anspruch 1, wobei,
beim Ausbilden des rippenartigen Abschnitts (3at),
das vorhergehende Material (3a) derart geschnitten wird, dass eine Länge des Stapelabschnitts (3d) größer ist als eine Weglänge des Materials von dem stromabwärtigen Ende des Wicklungsabschnitts (3t) des Materials zu einem stromabwärtigen Ende des anderen Wicklungsabschnitts (3t) des Materials,
wobei der Wicklungsabschnitt (3t) ein Abschnitt ist, der um die Eintrittsrolle (41 u) gewickelt ist,
wobei der andere Wicklungsabschnitt (3t) ein Abschnitt ist, der um die bewegliche Rolle (41m) gewickelt ist, die an der Referenzposition angeordnet ist.

3. Verfahren zum Verbinden eines Materials gemäß Anspruch 2, wobei
das Material in Förderrichtung gefördert wird, während das Material um die Eintrittsrolle (41u), die bewegliche Rolle (41m) und eine Austrittsrolle (41d) der Tänzereinheit (40) gewickelt wird, wobei,
nach dem Verbinden des nachfolgenden Materials (3f) und des vorhergehenden Materials (3a),
das Fördern des Materials, bei dem der verbundene Abschnitt (3j) und der Stapelabschnitt (3d) entlang der Förderrichtung nebeneinander vorgesehen sind, während das Material um die Eintrittsrolle (41u), die bewegliche Rolle (41m) und die Austrittsrolle (41d) gewickelt wird, andauert und wobei,
beim Ausbilden des rippenartigen Abschnitts (3at),
das vorhergehende Material (3a) derart geschnitten ist, dass die Gesamtlänge des verbundenen Abschnitts (3j) und des Stapelabschnitts (3d) größer als eine Weglänge des Materials von dem stromabwärtigen Ende des Wicklungsabschnitts (3t) des Materials durch die an der Referenzposition angeordnete bewegliche Rolle (41m) zu einem stromaufwärtigen Ende eines anderen Wicklungsabschnitts (3t) des Materials ist,
wobei der Wicklungsabschnitt (3t) ein Abschnitt ist, der um die Eintrittsrolle (41u) gewickelt ist,
wobei der andere Wickelabschnitt (3t) ein Abschnitt ist, der um die Ausgangsrolle (41d) gewickelt ist.

4. Verfahren zum Verbinden eines Materials gemäß Anspruch 3, wobei
beim Ausbilden des rippenartigen Abschnitts (3at),
das vorhergehende Material (3a) derart geschnitten wird, dass die Länge des Stapelabschnitts (3d) größer ist als eine Weglänge des Materials vom stromabwärtigen Ende des Wicklungsabschnitts (3t) des Materials über die an der Referenzposition angeordnete bewegliche Rolle (41m) zu einem stromabwärtigen Ende des anderen Wicklungsabschnitts (3t) des Materials,
der Wicklungsabschnitt (3t) ein Abschnitt ist, der um die Eintrittsrolle (41u) gewickelt ist,
wobei der andere Wickelabschnitt (3t) ein Abschnitt ist, der um die Austrittsrolle (41d) gewickelt ist.

5. Verfahren zum Verbinden eines Materials nach Anspruch 2, wobei
das Material in Förderrichtung gefördert wird, während das Material um die Eintrittsrolle (41u), die bewegliche Rolle (41m) und eine Austrittsrolle (41d) der Tänzereinheit (40) gewickelt wird, wobei,
nach dem Verbinden des nachfolgenden Materials (3f) und des vorhergehenden Materials (3a),
das Fördern des Materials, bei dem der verbundene Abschnitt (3j) und der Stapelabschnitt (3d) nebeneinander entlang der Förderrichtung vorgesehen sind, andauert, während das um die Einlaufrolle (41u), die bewegliche Rolle (41m) und die Auslaufrolle (41d) gewickelte Material, und wobei
beim Ausbilden des rippenartigen Abschnitts (3at),
das vorhergehende Material (3a) derart geschnitten ist, dass die Gesamtlänge des verbundenen Abschnitts (3j) und des Stapelabschnitts (3d) kleiner als eine Weglänge des Materials von dem stromabwärtigen Ende des Wicklungsabschnitts (3t) des Materials durch die an der Referenzposition angeordnete bewegliche Rolle (41m) zu einem stromaufwärtigen Ende eines anderen Wicklungsabschnitts (3t) des Materials ist,
wobei der Wicklungsabschnitt (3t) ein Abschnitt ist, der um die Eintrittsrolle (41u) gewickelt ist,
wobei der andere Wicklungsabschnitt (3t) ein Abschnitt ist, der um die Austrittsrolle (41d) gewickelt ist.

6. Verfahren zum Verbinden eines Materials gemäß einem der Ansprüche 1 bis 5, wobei,
beim Ausbilden des rippenartigen Abschnitts (3at),
das vorhergehende Material (3a) derart geschnitten ist, dass die Gesamtlänge des verbundenen Abschnitts (3j) und des Stapelabschnitts (3d) größer ist als eine Weglänge des Materials von einem stromaufwärtigen Ende des Wicklungsabschnitts (3t) des Materials zu dem stromaufwärtigen Ende des anderen Wicklungsabschnitts (3t) des Materials,
wobei der Wicklungsabschnitt (3t) ein Abschnitt ist, der um die Eintrittsrolle (41u) gewickelt ist,
wobei der andere Wicklungsabschnitt (3t) ein Abschnitt ist, der um die bewegliche Rolle (41m) gewickelt ist, die an der Referenzposition angeordnet ist.

7. Verfahren zum Verbinden eines Materials gemäß Anspruch 6, wobei,
beim Verbinden des nachfolgenden Materials (3f) mit dem geförderten vorhergehenden Material (3a),
das vorhergehende Material (3a) gegen das nachfolgende Material (3f) gedrückt wird, indem ein Anschlagelement aus einer Bereitschaftsposition in eine Anschlagsposition bewegt wird, in der das Anschlagelement an dem vorhergehenden Material (3a) anliegt, wobei,
nachdem der verbundene Abschnitt (3j) des zu transportierenden Materials die bewegliche Rolle (41m) erreicht hat,
das Anschlagelement von der Anschlagsposition in die Bereitschaftsposition zurückbewegt wird, und wobei,
beim Ausbilden des rippenartigen Abschnitts (3at),
das vorhergehende Material (3a) derart geschnitten ist, dass die Gesamtlänge des verbundenen Abschnitts (3j) und des Stapelabschnitts (3d) größer ist als eine Weglänge des Materials von einem stromabwärtigen Ende eines Anschlagabschnitts des Materials zu dem stromaufwärtigen Ende des anderen Wickelabschnitts (3t) des Materials,
wobei der Anschlagabschnitt ein Abschnitt ist, der an dem Anschlagelement anliegt,
wobei der andere Wickelabschnitt (3t) ein Abschnitt ist, der um die bewegliche Rolle (41m) gewickelt ist, die an der Referenzposition angeordnet ist.

8. Verfahren zum Verbinden eines Materials gemäß Anspruch 7, wobei,
nachdem der verbundene Abschnitt (3j) des zu fördernden Materials die bewegliche Rolle (41m) passiert hat,
das Anschlagelement von der Anschlagsposition in die Bereitschaftsposition zurückbewegt wird, und wobei,
beim Ausbilden des rippenartigen Abschnitts (3at),
das vorhergehende Material (3a) derart geschnitten ist, dass eine Länge des Stapelabschnitts (3d) größer ist als eine Weglänge des Materials von dem stromabwärtigen Ende des Anschlagsabschnitts des Materials zu dem stromaufwärtigen Ende des anderen Wicklungsabschnitts (3t) des Materials zu einem stromabwärtigen Ende des anderen Wicklungsabschnitts (3t) des Materials,
wobei der Anschlagabschnitt ein Abschnitt ist, der an dem Anschlagelement anliegt,
wobei der andere Wicklungsabschnitt (3t) ein Abschnitt ist, der um die bewegliche Walze (41m) gewickelt ist, die an der Referenzposition angeordnet ist.

9. Verfahren zum Verbinden eines Materials gemäß Anspruch 6, wobei,
beim Verbinden des nachfolgenden Materials (3f) mit dem geförderten vorhergehenden Material (3a),
das vorhergehende Material (3a) gegen das nachfolgende Material (3f) gedrückt wird, indem ein Anschlagelement aus einer Bereitschaftsposition in eine Anschlagsposition bewegt wird, in der das Anschlagelement an dem vorhergehenden Material (3a) anliegt, und wobei,
beim Ausbilden des rippenartigen Abschnitts (3at),
das vorhergehende Material (3a) derart geschnitten ist, dass die Gesamtlänge des verbundenen Abschnitts (3j) und des Stapelabschnitts (3d) kleiner als eine Weglänge des Materials von einem stromabwärtigen Ende eines Anschlagabschnitts des Materials zu dem stromaufwärtigen Ende des anderen Wicklungsabschnitts (3t) des Materials ist,
wobei der Anschlagabschnitt ein Abschnitt ist, der an dem Anschlagelement anliegt,
wobei der andere Wicklungsabschnitt (3t) ein Abschnitt ist, der um die bewegliche Walze (41m) gewickelt ist, die an der Referenzposition angeordnet ist.

10. Verfahren zum Verbinden eines Materials gemäß einem der Ansprüche 1 bis 9, wobei,
beim Verbinden des nachfolgenden Materials (3f) mit dem geförderten vorhergehenden Material (3a),
das vorhergehende Material (3a) gegen das nachfolgende Material (3f) gedrückt wird, indem ein Anschlagelement aus einer Bereitschaftsposition in eine Anschlagsposition bewegt wird, in der das Anschlagelement an dem vorhergehenden Material (3a) anliegt, und wobei
das vorhergehende Material (3a) mit dem an der Anschlagposition angeordneten Anschlagelement geschnitten wird.

11. Verfahren zum Verbinden eines Materials gemäß Anspruch 10, wobei,
nach dem Lösen des Anliegens des rippenartigen Abschnitts (3at) des auf dem Anschlagelement zu fördernden Materials,
das Anschlagelement von der Anschlagsposition in die Bereitschaftsposition zurückbewegt wird.

12. Verfahren zum Verbinden eines Materials gemäß einem der Ansprüche 1 bis 11, wobei,
beim Verbinden des nachfolgenden Materials (3f) mit dem geförderten vorhergehenden Material (3a),
das vorhergehende Material (3a) gegen das nachfolgende Material (3f) gedrückt wird, indem ein Anschlagelement aus einer Bereitschaftsposition in eine Anschlagsposition bewegt wird, in der das Anschlagelement an dem vorhergehenden Material (3a) anliegt, und wobei
das Anschlagelement ein Riemenelement ist, das rotierbar ist.

13. Verfahren zum Verbinden eines Materials gemäß Anspruch 12, wobei
das Riemenelement ist ein Element
das aufgrund von Rotationen einer ersten Rotationswalze und einer zweiten Rotationswalze rotiert, während das Riemenelement um die erste Rotationswalze und die zweite Rotationswalze gewickelt wird, und
das sich von der Bereitschaftsposition in die Anschlagsposition durch Schwenken um eine Schwenkachse bewegt,
wobei die Schwenkachse eine Mittelachse der ersten Rotationswalze ist,

14. Verfahren zum Verbinden eines Materials gemäß einem der Ansprüche 1 bis 13, wobei
das Material eine kontinuierliche Lage ist, die eine Faseranordnung ist,
wobei eine Oberfläche der kontinuierlichen Lage eine höhere Faserdichte aufweist als eine andere Oberfläche der kontinuierlichen Lage, und wobei,
beim Verbinden des nachfolgenden Materials (3f) mit dem geförderten vorhergehenden Material (3a),
das vorhergehende Material (3a) gegen das nachfolgende Material (3f) gedrückt wird, während ein Klebeelement zwischen dem vorhergehenden Material (3a) und dem nachfolgenden Material (3f) sandwichartig angeordnet ist, indem ein Anschlagelement aus einer Bereitschaftsposition in eine Anschlagsposition bewegt wird, in der das Anschlagelement an der einen Oberfläche des vorhergehenden Materials (3a) anliegt.

15. Verfahren zum Verbinden eines Materials gemäß einem der Ansprüche 1 bis 14, wobei
das Material eine kontinuierliche Lage ist, die eine Faseranordnung ist,
wobei eine Oberfläche der kontinuierlichen Lage eine höhere Faserdichte aufweist als eine andere Oberfläche der kontinuierlichen Lage, und wobei,
beim Ausbilden des rippenartigen Abschnitts (3at) durch Schneiden des vorhergehenden Materials (3a),
ein Schneidmesser von der einen Oberfläche eintritt.

16. Vorrichtung zum Zuführen eines Materials, das einem absorbierenden Artikel zugeordnet ist, umfassend:
eine Tänzereinheit (40), die eine Eintrittsrolle (41u) und eine bewegliche Rolle (41m) umfasst;
einen Transportabschnitt, der ausgebildet ist, um das Material in einer Förderrichtung zu fördern, während das Material um die Eintrittsrolle (41u) und die bewegliche Rolle (41m) gewickelt ist;
einen Materialverbindungsabschnitt, der ausgebildet ist, um ein nachfolgendes Material (3f) mit einem vorhergehenden Material (3a) zu verbinden, indem ein vorderer Endabschnitt des nachfolgenden Materials (3f) mit dem vorhergehenden Material (3a) verbunden wird,
wobei das vorhergehende Material (3a) das Material ist, das gefördert wird;
einen Schneidabschnitt, der ausgebildet ist, um einen rippenartigen Abschnitt (3at) des vorhergehenden Materials (3a) stromaufwärts in der Förderrichtung von einem verbundenen Abschnitt (3j) auszubilden, in dem das vorhergehende Material (3a) und das nachfolgende Material (3f) verbunden sind, wobei das Ausbilden durch Schneiden des vorhergehenden Materials (3a) an einer stromaufwärtigen Position von dem verbundenen Abschnitt (3j) ausgeführt wird, wobei das Fördern des Materials, in dem der verbundene Abschnitt (3j) und ein Stapelabschnitt (3d) nebeneinander entlang der Förderrichtung vorgesehen sind, von dem Transportabschnitt fortgeführt wird, während das Material um die Eintrittsrolle (41u) und die bewegliche Rolle (41m) gewickelt wird,
wobei der Stapelabschnitt (3d) ein Abschnitt ist, in dem der flossenartige Abschnitt (3at) und das nachfolgende Material (3f) gestapelt sind; und
einen Steuerabschnitt, der ausgebildet ist, um den Transport des Materials zu steuern, so dass die bewegliche Rolle (41m) an einer Referenzposition angeordnet ist,
wobei der Schneidabschnitt ausgebildet ist, um das vorhergehende Material (3a) derart zu schneiden, dass eine Gesamtlänge des verbundenen Abschnitts (3j) und des Stapelabschnitts (3d) größer als eine Weglänge des Materials von einem stromabwärtigen Ende eines Wicklungsabschnitts des Materials zu einem stromaufwärtigen Ende eines weiteren Wicklungsabschnitts (3t) des Materials ist,
wobei der Wicklungsabschnitt ein Abschnitt ist, der um die Eintrittsrolle (41 u) gewickelt ist, und
wobei der andere Wickelabschnitt (3t) ein Abschnitt ist, der um die bewegliche Rolle (41m) gewickelt ist, die an der Referenzposition angeordnet ist.

## Revendications

1. Procédé d'épissage d'un matériau associé à un article absorbant, comprenant :
le transport du matériau dans une direction de transport tandis que le matériau est enroulé autour d'un rouleau d'entrée (41 u) et d'un rouleau de déplacement (41m),
le rouleau d'entrée (41u) et le rouleau de déplacement (41m) étant inclus dans une unité de rouleau danseur (40) ;
l'épissage d'un matériau suivant (3f) avec un matériau précédent (3a) par liaison d'une portion d'extrémité avant du matériau suivant (3f) avec le matériau précédent (3a),
le matériau précédent (3a) étant le matériau qui est transporté ;
la formation d'une portion de type ailette (3at) du matériau précédent (3a) en amont dans la direction de transport depuis une portion liée (3j) où le matériau précédent (3a) et le matériau suivant (3f) sont liés,
la formation étant réalisée en coupant le matériau précédent (3a) à une position en amont de la portion liée (3j) ;
la poursuite du transport du matériau dans lequel la portion liée (3j) et une portion d'empilement (3d) sont prévues côte à côte suivant la direction de transport, tandis que le matériau est enroulé autour du rouleau d'entrée (41u) et du rouleau de déplacement (41m),
la portion d'empilement (3d) étant une portion où la portion de type ailette (3at) et le matériau suivant (3f) sont empilés ; et
la commande du transport du matériau de telle sorte que le rouleau de déplacement (41m) est situé à une position de référence,
dans la formation de la portion de type ailette (3at),
le matériau précédent (3a) est coupé de telle sorte qu'une longueur totale de la portion liée (3j) et de la portion d'empilement (3d) est plus grande qu'une longueur de trajet du matériau depuis une extrémité aval (P3) d'une portion d'enroulement (3t) du matériau jusqu'à une extrémité amont (P4) d'une autre portion d'enroulement du matériau,
la portion d'enroulement (3t) étant une portion enroulée autour du rouleau d'entrée (41u),
l'autre portion d'enroulement (3t) étant une portion enroulée autour du rouleau de déplacement (41m) situé à la position de référence.

2. Procédé d'épissage d'un matériau selon la revendication 1, dans lequel
dans la formation de la portion de type ailette (3at),
le matériau précédent (3a) est coupé de telle sorte qu'une longueur de la portion d'empilement (3d) est plus grande qu'une longueur de trajet du matériau depuis l'extrémité aval de la portion d'enroulement (3t) du matériau jusqu'à une extrémité aval de l'autre portion d'enroulement (3t) du matériau,
la portion d'enroulement (3t) étant une portion enroulée autour du rouleau d'entrée (41u),
l'autre portion d'enroulement (3t) étant une portion enroulée autour du rouleau de déplacement (41m) situé à la position de référence.

3. Procédé d'épissage d'un matériau selon la revendication 2, dans lequel
le matériau est transporté dans la direction de transport tandis que le matériau est enroulé autour du rouleau d'entrée (41u), du rouleau de déplacement (41m), et du rouleau de sortie (41d) de l'unité de rouleau danseur (40),
après épissage du matériau suivant (3f) et du matériau précédent (3a),
le transport du matériau dans lequel la portion liée (3j) et la portion d'empilement (3d) sont prévues côte à côte suivant la direction de transport se poursuit tandis que le matériau est enroulé autour du rouleau d'entrée (41u), du rouleau de déplacement (41m) et du rouleau de sortie (41d), et
dans la formation de la portion de type ailette (3at),
le matériau précédent (3a) est coupé de telle sorte que la longueur totale de la portion liée (3j) et de la portion d'empilement (3d) est plus grande qu'une longueur de trajet du matériau depuis l'extrémité aval de la portion d'enroulement (3t) du matériau, à travers le rouleau de déplacement (41m) situé à la position de référence, jusqu'à une extrémité amont d'une autre portion d'enroulement (3t) du matériau,
la portion d'enroulement (3t) étant une portion enroulée autour du rouleau d'entrée (41u),
l'autre portion d'enroulement (3t) étant une portion enroulée autour du rouleau de sortie (41d).

4. Procédé d'épissage d'un matériau selon la revendication 3, dans lequel
dans la formation de la portion de type ailette (3at),
le matériau précédent (3a) est coupé de telle sorte que la longueur de la portion d'empilement (3d) est plus grande qu'une longueur de trajet du matériau depuis l'extrémité aval de la portion d'enroulement (3t) du matériau, à travers le rouleau de déplacement (41m) situé à la position de référence, jusqu'à une extrémité aval de l'autre portion d'enroulement (3t) du matériau,
la portion d'enroulement (3t) étant une portion enroulée autour du rouleau d'entrée (41u),
l'autre portion d'enroulement (3t) étant une portion enroulée autour du rouleau de sortie (41d).

5. Procédé d'épissage d'un matériau selon la revendication 2, dans lequel
le matériau est transporté dans la direction de transport tandis que le matériau est enroulé autour du rouleau d'entrée (41u), du rouleau de déplacement (41m), et d'un rouleau de sortie (41d) de l'unité de rouleau danseur (40),
après épissage du matériau suivant (3f) et du matériau précédent (3a),
le transport du matériau dans lequel la portion liée (3j) et la portion d'empilement (3d) sont fournies côte à côte suivant la direction de transport se poursuit tandis que le matériau est enroulé autour du rouleau d'entrée (41u), du rouleau de déplacement (41m) et du rouleau de sortie (41d), et
dans la formation de la portion de type ailette (3at),
le matériau précédent (3a) est coupé de telle sorte que la longueur totale de la portion liée (3j) et de la portion d'empilement (3d) est plus petite qu'une longueur de trajet du matériau depuis l'extrémité aval de la portion d'enroulement (3t) du matériau, à travers le rouleau de déplacement (41m) situé à la position de référence, jusqu'à une extrémité amont d'une autre portion d'enroulement (3t) du matériau,
la portion d'enroulement (3t) étant une portion enroulée autour du rouleau d'entrée (41 u),
l'autre portion d'enroulement (3t) étant une portion enroulée autour du rouleau de sortie (41d).

6. Procédé d'épissage d'un matériau selon l'une quelconque des revendications 1 à 5, dans lequel
dans la formation de la portion de type ailette (3at),
le matériau précédent (3a) est coupé de telle sorte que la longueur totale de la portion liée (3j) et de la portion d'empilement (3d) est plus grande qu'une longueur de trajet du matériau depuis une extrémité amont de la portion d'enroulement (3t) du matériau jusqu'à une extrémité amont de l'autre portion d'enroulement (3t) du matériau,
la portion d'enroulement (3t) étant une portion enroulée autour du rouleau d'entrée (41u),
l'autre portion d'enroulement (3t) étant une portion enroulée autour du rouleau de déplacement (41m) situé à la position de référence.

7. Procédé d'épissage d'un matériau selon la revendication 6, dans lequel lors de la liaison du matériau suivant (3f) avec le matériau précédent (3a) qui est transporté,
le matériau précédent (3a) est appuyé contre le matériau suivant (3f) en déplaçant un organe de butée depuis une position en attente jusqu'à une position de butée où l'organe de butée bute contre le matériau précédent (3a),
après que la portion liée (3j) du matériau qui est transporté a atteint le rouleau de déplacement (41m),
l'organe de butée est reculé de la position de butée à la position d'attente, et
dans la formation de la portion de type ailette (3at),
le matériau précédent (3a) est coupé de telle sorte que la longueur totale de la portion liée (3j) et de la portion d'empilement (3d) est plus grande qu'une longueur de trajet du matériau depuis une extrémité aval d'une portion de butée du matériau jusqu'à l'extrémité amont de l'autre portion d'enroulement (3t) du matériau,
la portion de butée étant une portion qui bute contre l'organe de butée,
l'autre portion d'enroulement (3t) étant une portion enroulée autour du rouleau de déplacement (41m) situé à la position de référence.

8. Procédé d'épissage d'un matériau selon la revendication 7, dans lequel après que la portion liée (3j) du matériau qui est transporté a dépassé le rouleau de déplacement (41m),
l'organe de butée est reculé de la position de butée à la position d'attente, et
dans la formation de la portion de type ailette (3at),
le matériau précédent (3a) est coupé de telle sorte qu'une longueur de la portion d'empilement (3d) est plus grande qu'une longueur de trajet du matériau depuis l'extrémité aval de la portion de butée du matériau jusqu'à l'extrémité amont de l'autre portion d'enroulement (3t) du matériau jusqu'à une extrémité aval de l'autre portion d'enroulement (3t) du matériau,
la portion de butée étant une portion qui bute contre l'organe de butée,
l'autre portion d'enroulement (3t) étant une portion enroulée autour du rouleau de déplacement (41m) situé à la position de référence.

9. Procédé d'épissage d'un matériau selon la revendication 6, dans lequel lors de la liaison du matériau suivant (3f) avec le matériau précédent (3a) qui est transporté,
le matériau précédent (3a) est appuyé contre le matériau suivant (3f) en déplaçant un organe de butée d'une position d'attente à une position de butée où l'organe de butée bute contre le matériau précédent (3a), et
dans la formation de la portion de type ailette (3at),
le matériau précédent (3a) est coupé de telle sorte que la longueur totale de la portion liée (3j) et de la portion d'empilement (3d) est plus petite qu'une longueur de trajet du matériau depuis une extrémité aval d'une portion de butée du matériau jusqu'à l'extrémité amont de l'autre portion d'enroulement (3t) du matériau,
la portion de butée étant une portion qui bute contre l'organe de butée,
l'autre portion d'enroulement (3t) étant une portion enroulée autour du rouleau de déplacement (41m) situé à la position de référence.

10. Procédé d'épissage d'un matériau selon l'une quelconque des revendications 1 à 9, dans lequel
lors de la liaison du matériau suivant (3f) avec le matériau précédent (3a) qui est transporté,
le matériau précédent (3a) est appuyé contre le matériau suivant (3f) en déplaçant un organe de butée d'une position d'attente à une position de butée où l'organe de butée bute contre le matériau précédent (3a), et
le matériau précédent (3a) est coupé avec l'organe de butée situé à la position de butée.

11. Procédé d'épissage d'un matériau selon la revendication 10, dans lequel
après relâchement de la butée de la portion de type ailette (3at) du matériau qui est transporté sur l'organe de butée,
l'organe de butée est reculé de la position de butée à la position d'attente.

12. Procédé d'épissage d'un matériau selon l'une quelconque des revendications 1 à 11, dans lequel
lors de la liaison du matériau suivant (3f) avec le matériau précédent (3a) qui est transporté,
le matériau précédent (3a) est appuyé contre le matériau suivant (3f) en déplaçant un organe de butée d'une position d'attente à une position de butée où l'organe de butée bute contre le matériau précédent (3a), et
l'organe de butée est un organe courroie capable de tourner.

13. Procédé d'épissage d'un matériau selon la revendication 12, dans lequel l'organe courroie est un organe
qui tourne en raison de rotations d'un premier galet de rotation et d'un second galet de rotation tandis que l'organe courroie est enroulé autour du premier galet de rotation et du second galet de rotation, et
qui se déplace de la position d'attente à la position de butée en oscillant autour d'un axe d'oscillation,
l'axe d'oscillation étant un axe central du premier galet de rotation.

14. Procédé d'épissage d'un matériau selon l'une quelconque des revendications 1 à 13, dans lequel
le matériau est une feuille continue qui est un ensemble de fibres,
une surface de la feuille continue a une densité de fibre plus élevée qu'une autre surface de la feuille continue, et
lors de la liaison du matériau suivant (3f) avec le matériau précédent (3a) qui est transporté,
le matériau précédent (3a) est appuyé contre le matériau suivant (3f) tandis qu'un organe adhésif est enserré entre le matériau précédent (3a) et le matériau suivant (3f) en déplaçant un organe de butée d'une position d'attente à une position de butée où l'organe de butée bute contre la surface du matériau précédent (3a).

15. Procédé d'épissage d'un matériau selon l'une quelconque des revendications 1 à 14, dans lequel
le matériau est une feuille continue qui est un ensemble de fibres,
une surface de la feuille continue a une densité de fibre plus élevée qu'une autre surface de la feuille continue, et
dans la formation de la portion de type ailette (3at) en coupant le matériau précédent (3a),
une lame de dispositif coupant entre depuis la surface.

16. Dispositif d'apport d'un matériau associé à un article absorbant, comprenant :
une unité de rouleau danseur (40) incluant un rouleau d'entrée (41u) et un rouleau de déplacement (41m) ;
une section de transport conçue pour transporter le matériau dans une direction de transport tandis que le matériau est enroulé autour du rouleau d'entrée (41u) et du rouleau de déplacement (41m) ;
une section d'épissage de matériau conçue pour épisser un matériau suivant (3f) avec un matériau précédent (3a) en liant une portion d'extrémité avant du matériau suivant (3f) avec le matériau précédent (3a),
le matériau précédent (3a) étant le matériau qui est transporté ;
une section de coupe qui est conçue pour former une portion de type ailette (3at) du matériau précédent (3a) en amont dans la direction de transport depuis une portion liée (3j) où le matériau précédent (3a) et le matériau suivant (3f) sont liés, moyennant quoi la formation est réalisée en coupant le matériau précédent (3a) à une position en amont de la portion liée (3j), moyennant quoi le transport du matériau dans lequel la portion liée (3j) et une portion d'empilement (3d) sont fournies côte à côte suivant la direction de transport se poursuit par la section de transport tandis que le matériau est enroulé autour du rouleau d'entrée (41u) et du rouleau de déplacement (41m),
la portion d'empilement (3d) étant une portion où la portion de type ailette (3at) et le matériau suivant (3f) sont empilés ; et
une section de commande conçue pour commander le transport du matériau de telle sorte que le rouleau de déplacement (41m) est situé à une position de référence,
la section de coupe étant conçue pour couper le matériau précédent (3a) de telle sorte qu'une longueur totale de la portion liée (3j) et de la portion d'empilement (3d) est plus grande qu'une longueur de trajet du matériau depuis une extrémité aval d'une portion d'enroulement du matériau jusqu'à une extrémité amont d'une autre portion d'enroulement (3t) du matériau, moyennant quoi
la portion d'enroulement est une portion enroulée autour du rouleau d'entrée (41u), et l'autre portion d'enroulement (3t) est une portion enroulée autour du rouleau de déplacement (41m) situé à la position de référence.
